# EUROPEAN PATENT SPECIFICATION

(11) **EP 4 565 573 B1**
(45) Date of publication and mention of the grant of the patent: **15.04.2026**
(21) Application number: 23754253.5
(22) Date of filing: 07.08.2023
(51) Int. Cl.: C07D 311/80, C07C 37/16, A61P 25/04, A61K 31/00

(54) **CANNABINOID SYNTHESIS STARTING OUT FROM OLIVETOL AND TERPENE IN DICHLOROMETHANE WITH FECL3 * 6H2O AS CATALYST**
CANNABINOIDSYNTHESE AUSGEHEND VON OLIVETOL UND TERPEN IN DICHLOROMETHAN MIT FECL3 * 6H2O ALS KATALYSATOR
SYNTHÈSE DE CANNABINOÏDE À PARTIR D'OLIVÉTOL ET TERPÈNE DANS DE DICHLOROMÉTHANE AVEC FECL3 * 6H2O EN TANT QUE CATALYSEUR

(30) Priority: 05.08.2022 ES 202230730
(43) Date of publication of application: 11.06.2025
(73) Proprietor: Salud & Semillas, S.L., 29005 Malaga (ES)
(72) Inventor: LÓPEZ ROMERO, Juan Manuel, 29005 MALAGA (ES); MOYA UTRERA, Federico, 29005 MALAGA (ES); ROMERO CARRASCO, Antonio, 29005 MALAGA (ES); SARABIA GARCÍA, Francisco, 29005 MALAGA (ES)
(74) Representative: Isern Patentes y Marcas S.L.
(86) International application number: PCT/EP2023/071806
(87) International publication number: WO 2024/028516

(56) References cited:
- WO-A2-2007/041167
- CN-A- 113 372 196
- US-A1- 2022 144 737
- US-A1- 2022 204 431

## Description

### Field of the invention

The present invention relates to the field of the synthesis of natural products by chemical processes. More specifically, it relates to a method for the synthesis of cannabidiol (CBD) and tetrahydrocannabinol (THC).

### Background of the invention

Widely used since ancient times in traditional Asian medicine, particularly in India, the *Cannabis sativa* plant, belonging to the Cannabaceae family, has gained great interest and expectation in recent years, both in scientific and social circles, mainly motivated by the biological action and therapeutic value of cannabinoids. These natural products are unique and exclusive to this plant and can be classified as C21 terpeno-phenolic compounds, mainly concentrated in the flower.

Among the 483-568 identified compounds of this plant, among which are derivatives of benzoic acids, cinnamic acids, flavonoids, lignans, stilbenes or terpenes, about 66 have been characterized as cannabinoids (other authors even estimate that the number of cannabinoids may even amount to 113, to which about 120 terpenes must be added), and the most important ones are:
- Δ⁹-tetrahydrocannabinol (9-THC, 1);
- cannabinol (CBN, 2);
- cannabichromene (CBC, 3),
- cannabigerol (CBG, 4);
- cannabinodiol (CBND, 5);
- cannabidiol (CBD, 8).

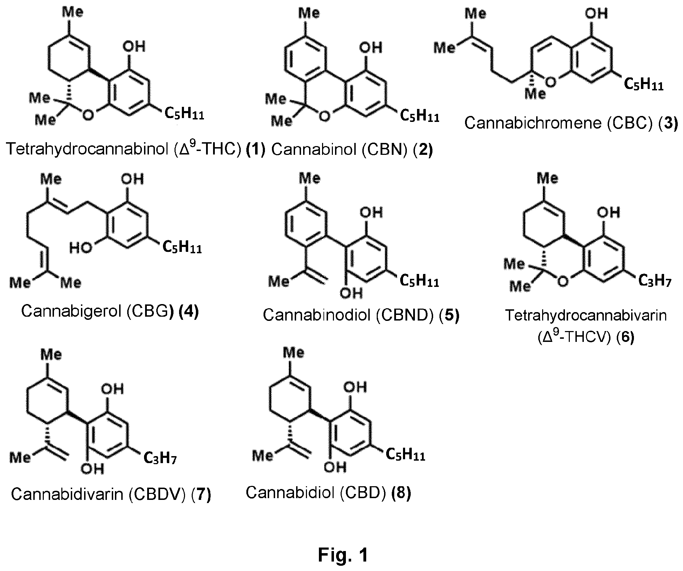

These compounds are represented in Fig. 1, in which a number is assigned for each one of them that coincides with the one indicated after the abbreviations of the previous list and that will be maintained throughout the present memory. This method of referring to the compounds with the number assigned to them in the figures will be followed with other compounds that are represented in subsequent figures and will be used individually, not including the name of the compound, when the compound is not commonly known by a common short name and/or when, for clarity of explanation, it is preferable to refer to it by its generic name and include its specific numbering behind it.

Definitely, the cannabinoid that focuses the greatest interest from the therapeutic point of view, besides being the most abundant, is cannabidiol (CBD, 8) (Mechoulam *et al.,* 2007). Its name according to the IUPAC systematic nomenclature is: (2-[(1R,6R)-6-isopropenyl-3-methylcyclohex-2-en-1-yl]-5-pentylbenzene-1,3-diol, and its formula is the following: 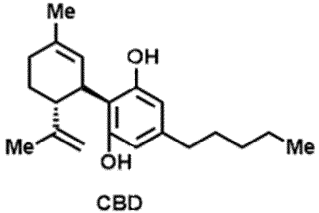 Δ⁹-THC (1) or, simply, THC, is also quite well known and is often cited in connection with *Cannabis sativa,* because it is responsible for the psychotropic effects of the plant. Its name according to the IUPAC systematic nomenclature is: tetrahydro-6,6,9-trimethyl-3-pentyl-6H-dibenzene[b,d]pyran-1-ol, and its formula is the following:

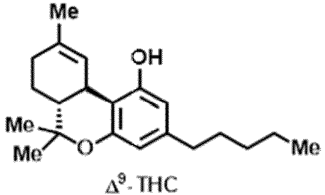

All these cannabinoids are in their acid form, which is how they are originally biosynthesized in the plant, but they are easily decarboxylated into their neutral forms, either by the action of heat, plant storage or during extraction and isolation processes.

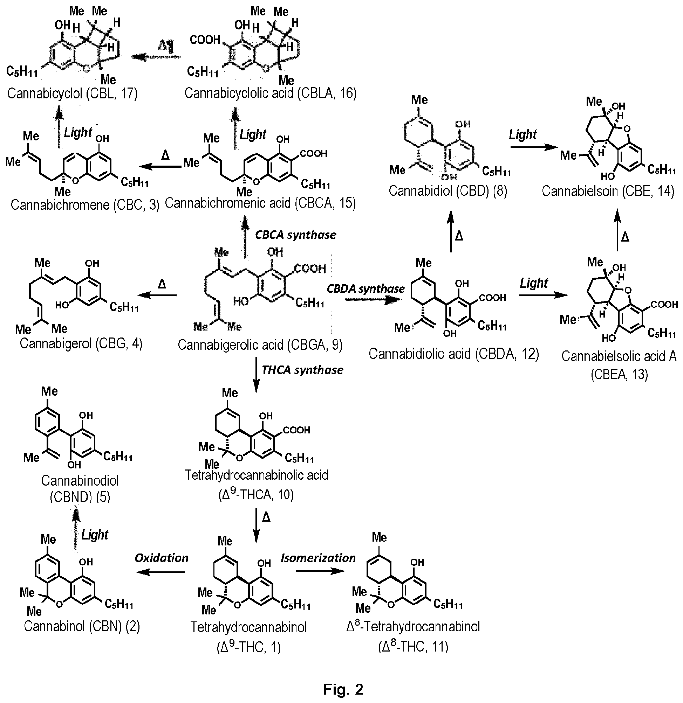

In that sense, the biosynthesis of all these compounds starts from cannabigerolic acid (CBGA, 9) from which the most important derivatives, such as Δ⁹-tetrahydrocannabinolic (Δ⁹-THCA, 10), cannabidiolic (CBDA, 12) and cannabichromenic (CBCA, 15) acids originate. These derivatives, through heat, irradiation or the oxidative action of air, can be transformed into a wide range of derivatives, among which the decarboxylated forms CBC, THC or CBD (Fig. 2) stand out.

According to the concentration of cannabinoids present in the plant, 5 chemotypes have been established, which are: type I, when the THCA/CBDA ratio is much higher than 1, type II, when this ratio is in the range of 0.5 to 2, type III, when the THCA/CBDA ratio is less than 1, type IV, when CBGA is the main cannabinoid, and type V, when the general level of cannabinoid content is extremely low.

Within the varieties obtained by genetic modification, which are the ones that will define the chemotype of the variety of Cannabis, strains with more than 15% CBD and less than 1% THC have recently been developed.

There is evidence that the first successful extraction of the active ingredients of this plant was carried out by Schlesinger in 1840 and, since then, there have been numerous studies for the isolation and purification of cannabinoids present in the plant. This extraction operation represents a process of some complexity and difficulty, mainly due to the sensitivity of this operation to multiple variables such as temperature and nature of the extracting solvents. The conventional method of extracting these natural products is based on the use of the Soxhlet method (the extraction method in which the solvent is heated to reflux and an amount of it is recirculated to the chamber where the material on which the extraction is performed is located, allowing to dissolve a large amount of material) and subsequent distillation of the extracts, with the drawbacks of long operating times and the large amounts of solvents required.

Given this, alternatives have been developed to improve and overcome these drawbacks, such as methods based on the use of supercritical CO₂, high pressure liquid extractions, use of microwaves, ultrasound, by means of pulsed electric fields, centrifugal partition chromatography and even enzymes. Despite this entire range of alternatives for the extraction of cannabinoids, only those based on supercritical CO₂ or high-pressure volatile solvents have been able to be developed on a pilot plant or industrial scale. Recent studies of cannabinoid production by means of *in vitro* cultures and by means of using genetically modified varieties must be added to the above processes.

The importance of purifying cannabinoids is based on the fact that, after considerable debate as to whether the medical benefits of cannabis were derived from the use of the mixture of these cannabinoids, it has been shown that the therapeutic benefits are greater when treated with pure cannabinoids (Fischedick *et al.,* 2010). In fact, the alleged synergy of cannabinoid products has been the cause of considerable skepticism. Conversely, the therapeutic value of certain combinations of pure cannabinoids with terpenes has been demonstrated, such as the combination of CBD with limonene, linalool and pinene for the treatment of anxiety, or with caryophyllene, linalool and myrcene for the treatment of sleep disorders, or the combination of cannabigerol with pinene as an antiseptic (Russo *et al.,* 2018).

As for the quantification of cannabinoids from extracts of a plant, the recent development of a quantification methodology by means of the NMR technique must be added to the widely developed and established conventional methods, based on the use of gas chromatography or HPLC techniques, coupled or not to mass spectrometry.

As an alternative to isolating cannabinoids from their natural sources, chemical synthesis to access these products, mainly CBD and THC, represents a valid pathway for their production on an industrial scale (Lago-Fernández *et al.,* 2017).

Following the structural determination of CBD by Adams *et al.* In 1940, there have been numerous reported syntheses of this natural product. In that sense, the first synthesis was published by Mechoulam in 1965 using citral A as a terpene derivative and obtaining the racemic mixture of CBD in what could be considered a biomimetic synthesis of this cannabinoid (Mechoulam *et al.,* 1965).

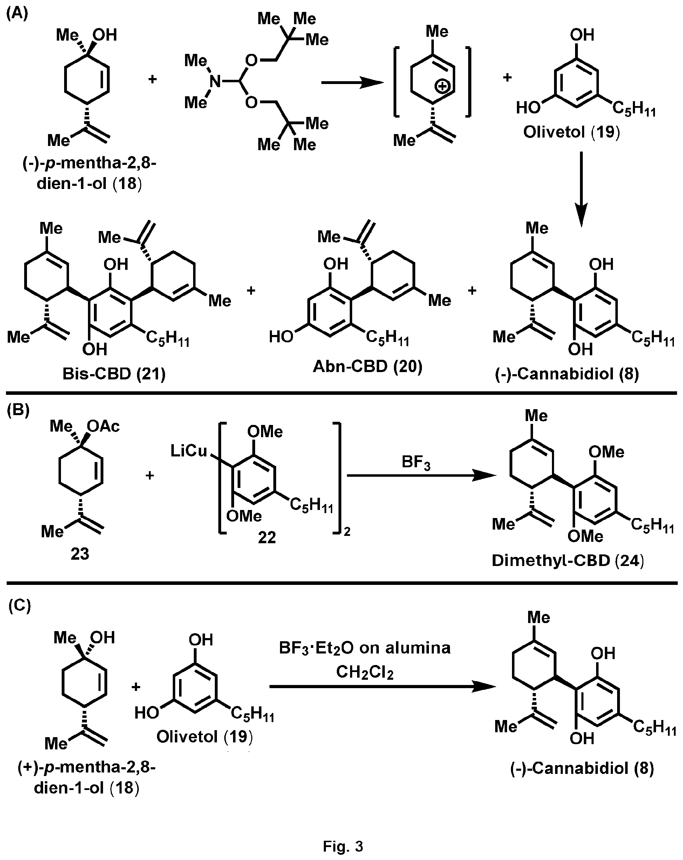

This synthesis was followed by the first stereoselective synthesis of CBD due to Petrzilka (Petrzilka *et al.,* 1967; Petrzilka *et al.,* 1969); starting from the terpene Δ-2,8-menthadien-1-ol (18), from which the corresponding carbocation was formed by reaction with N,N-dimethylformamide dineopentylacetal, and which reacted with olivetol (19) in an electrophilic aromatic substitution (EAS) reaction to form 25% (-)-CBD (8), but accompanied by 35% of the so-called abnormal-CBD, (abn-CBD, 20) and 5% of the disubstituted Bis-CBD derivative (21) and recovering up to 30% of the starting olivetol. This synthesis method is depicted in Scheme 1 below, as well as in Panel A of Fig. 3. Although the result was poor in terms of chemical yield and regioselectivity, the bases of what was a direct synthesis of CBD were established through the EAS reaction of olivetol.

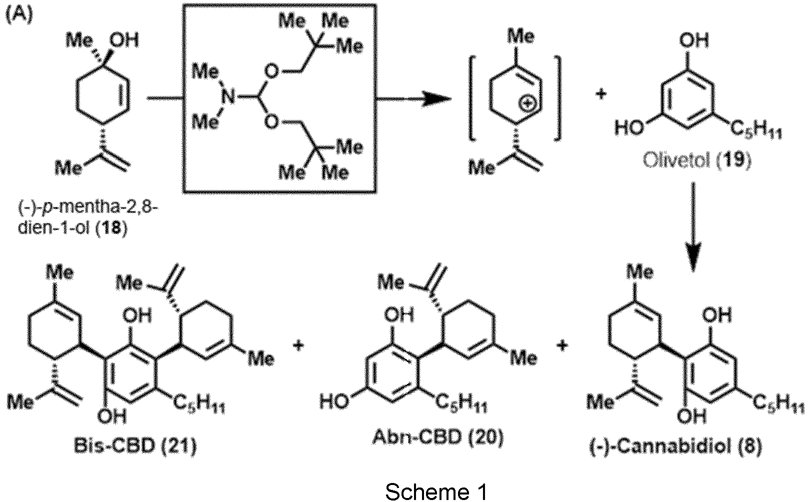

To that end, subsequent contributions have been based on this strategy, but with different reaction conditions to generate the corresponding electrophilic species, in the form of the carbocation of the terpene fragment. In that sense, the regioselectivity problem was addressed by Rickards *et al.* in 1984 by means of the formation of the copper derivative of olivetol 22, after lithiation, and reaction with the terpene derivative 23 catalyzed with BF₃ (Rickards *et al.,* 1984) (see Scheme 2 below, as well as Fig. 3, panel B).

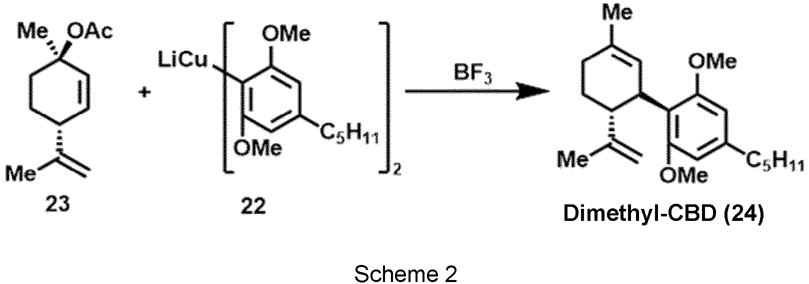

A year later, Mechoulam publishes the direct synthesis of CBD (Mechoulam *et al.,* 1965) by reaction of terpene 25 and olivetol using BF3 supported on alumina to obtain CBD with a 51% yield and minimizing the formation of abn-CBD by up to 12%. The process is depicted below in Scheme 3, as well as in panel C of Fig. 3; in both cases, t^{a} indicates room temperature.

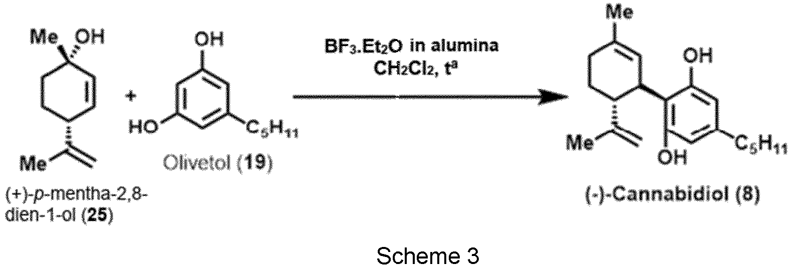

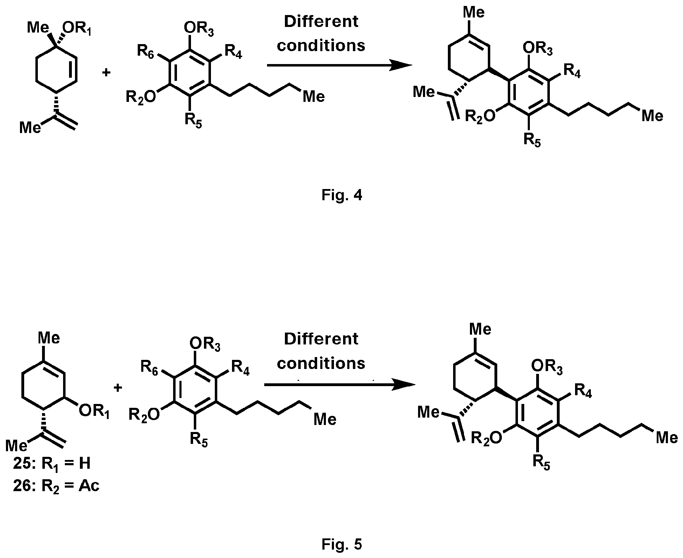

From then on, different Lewis acids have been tested that have not improved the results of Mechoulam, and only when olivetol has been modified by blocking positions 4 and 6, or position 4 of the aromatic system, better results have been achieved, but with the drawback of having to remove these auxiliary groups in additional steps. All these methods are represented in a generalized way in Scheme 4, also re produced in Fig. 4; in both cases, R₁, R₂, R₃, R₄, R₅ and R₆ may correspond to the radicals indicated in the corresponding column of Table 1 below, olivetol corresponding to the case in which all of them are H.

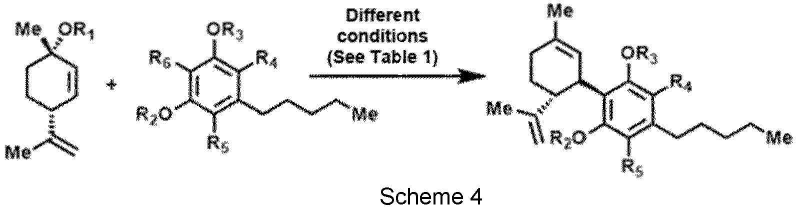

In that sense, the aforementioned Table 1 summarizes the contributions to CBD synthesis by direct coupling, presented in increasing temporal order, including the reaction conditions and the values of the radicals included in Scheme 4.

**Table 1. CBD synthesis timeline to the present**

| **Year** | **Author/ Application** | **Yield** | **Conditions** | **R₁** | **R₂** | **R₃** | **R₄** | **R₅** | **R₆** | **Conversion to CBD** |
|---|---|---|---|---|---|---|---|---|---|---|
| 1967 | Petrzilka *et al.* | 25% | Me₂NCH(OCH₂C(CH₃)₃)₂, DCM, t^{a}, 63h | H | H | H | H | H | H | 25% |
| 1969 | Petrzilka, T *et al.* | 7-24% | Mild protic acids and ZnCl2 | H | H | H | H | H | H | 7-24% |
| 1984 | Rickards and Ronneberg | 78 % | BF3.OEt2 3.5 eq, MgSO4, DCM, -76 °C, 5h | Ac | Me | Me | H | H | LiCu | 78 % |
| 1985 | Mechoulam *et al.* | 51 % | BF₃.OEt₂ Al₂O₃, MgSO₄, DCM, 40 °C, 10s | H | H | H | H | H | H | 51 % |
| 2005 | Burdick et al. (US2007093665) | 44 % | Sc(OTf)₃, MgSO₄, DCM, 10 °C, 3h | H | H | H | CO₂Et | H | H | 44 % |
| 2006 | Gutman et al. (WO2006053766) | 22 % | ZnCl₂, water and DCM, 1h, 40 °C | H | H | H | H | H | H | 22 % |
| 2007 | Burdick et al. (WO2007041167) | 44 % | Lewis acid triflates, DCM, 60 °C | H | H | H | CO₂Et | H | H | 58 % |
| 2009 | Stinchcomb et al. (WO2009018389) | 24 % | p-TsOH, benzene, 2.5h reflux | H | H | H | H | H | H | 24 % |
| 2009 | C. Steup, T Herkenroth (US2010298579) | 80 % | BF₃.OEt₂, MgSO₄, DCM, -76 °C, 5h | H | H | H | CO₂Me | H | H | >90 % |
| 2017 | Dialler et al. (WO2017011210) | 90 % | p-TsOH.H2O, DCM, - 35 °C at t^{a} overnight | H | H | H | Br | Br | H | 90 % |
| 2018 | Chen et al. (CN109734591) | 44 % | Sc(OTf)3, MgSO4, DCM, 10 °C, 3h | H | H | H | CO₂Et | H | H | 78 % |
| 2019 | Bencivenga et al. (WO2019046806) | - | Zn(OTf)₂ | H | H | H | H | H | H | - |
| 2020 | Zuhse et al. (DE102019104563) | 99 % | P-TsOH | H | H | H | Br | Br | H | 20 % |
| 2020 | Hallow et al. (WO2020051371) | 95 % | Protic acid catalysts, DCE | H | H | H | Br | Br | H | 86 % |
| 2020 | Porco et al. (US2020325091) | - | Lewis acids supported on resins or protic acids Continuous Flow | H | H | H | H | H | H | - |
| 2021 | Anand et al. (WO2021181420) | 40 % | AgNTf₂, DCE, 4h | H | H | H | H | H | H | 40 % |
| 2021 | Bloemendal *et al.* | 26% | Silica-BF₃ (Flow chemistry) | H | H | H | H | H | H | 26 % |
| 2022 | Zhang et al. (CN113896616) | 50-60 % | Protic acids, Lewis acids and acid resins | Ether or ester | Ester Ester | | H | H | H | 40-60 % |

Abbreviations: t^{a}: room temperature; DCM: dichloromethane (CH₂Cl₂); p-TsOH: p-toluene sulfonic acid; Sc(OTf)₃: scandium triflate; Tf: triflate (CF₃SO₂); DCE: dichloroethane (C₂H₂Cl₂);
As an alternative to the use of terpenes 18, 23 or 24, which, although commercially accessible, are very expensive for possible use on an industrial scale, some syntheses using derivatives 25 and 26, which are easily obtained from a much less expensive terpene, limonene, have been described. Similar to the syntheses in Table 1, starting from these derivatives by coupling with olivetol in EAS (electrophilic aromatic substitution) reactions, cannabidiol has been obtained, although with more modest results with respect to the previous reactions in terms of chemical yield and regioselectivity, as described in the publications indicated in Table 2. Said table appears after Scheme 5 that can be seen below, which, similarly to Fig. 5, represents these synthesis methods in a generalized way, including possible radicals R₁, R₂, R₃, R₄, R₅ and R₆ the possible values of which are indicated in the corresponding column of Table 2.

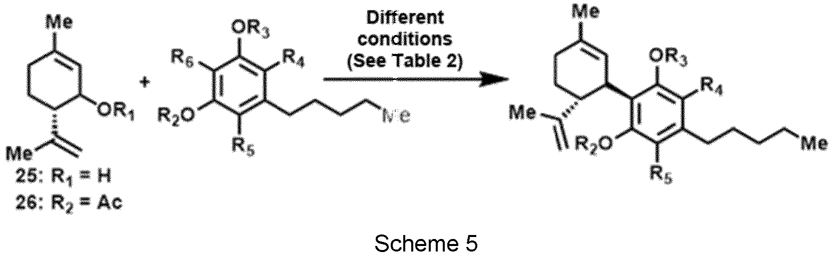

**Table 2. CBD synthesis from limonene derivatives 25 (R₁=H) or 26 (R₂=Ac)**

| **Year** | **Author/ Application** | **Yield** | **Conditions** | **R₁** | **R₂** | **R₃** | **R₄** | **R₅** | **R₆** | **Conversion to CBD** |
|---|---|---|---|---|---|---|---|---|---|---|
| 1972 | Cardillo *et al.* | 10 % | Oxalic acid, 20 °C, 3 days | H | H | H | H | H | H | 46 % |
| 2020 | Palmieri et al. (WO2020099283) | 51 % | BF₃.OEt₂, DCM, continuous flow | Ac | H | H | H | H | H | 47 % |
| 2020 | Shen et al. (CN111943813) | 50 % | BF₃ | H | H | H | H | H | H | 48 % |
| 2021 | Chirchiù *et al.* | 55 % | BF₃.OEt₂, DCM, continuous flow | Ac | H | H | H | H | H | 49 % |

### Abbreviation: DCM: dichloromethane (CH₂Cl₂);

In the same manner, the use of these terpene derivatives has been extended to other aromatic systems other than olivetol, with similar drawbacks (Dethe *et al.,* 2015; Gong *et al.,* 2020; Schultz *et al.,* 2018; Kobayashi *et al,* 2006; Bailey *et al.,* 2018).

More recent synthesis methods have not achieved to overcome such drawbacks. Thus, for instance, Bencivenga et al., in application US2022144737A1, have proposed a method for synthetically making cannabidiol and obtaining a product free of harmful and/or undesired impurities. The proposed method is similar to the one already provided by the same author and others in international application WO2019046806: synthesizing CBD using olivetol and (+)-menthadienol as suitable starting materials and using as catalyst a Lewis acid. As defined in US2022144737, Lewis acid catalysts are said to include, typically, a metal selected from aluminium, boron, silicon, tin, titanium, zinc, zirconium, iron or copper; although ZnCl₂, BF₃, SnCl₂, AlCl₃, Zn(OTf)₃ and Cu(OTf)₂ are mentioned as exemplary Lewis catalysts, only Zn(OTf)₃ is used in the Examples of the application. In some embodiments, the staring material is reacted using a solvent-based method wherein one or more of the starting materials are diluted in a diluting solvent and then mixed together; toluene, heptane and 1,2-dichloroethane are the specific solvents used in the Examples of CBD synthesis, although other possible solvents are mentioned as suitable for the method, such as other aliphatic solvents, aromatic solvents and halogenated solvents as dichloromethane, 1,2-dichloroethene, 1,1-dichloroethane, 1,1-dichloroethene, chlorobenzene and the like.

In order to obtain substantially pure cannabidiol, what is proposed in US2022144737A1 is to perform one or more steps additional to crude cannabidiol formation, such as an aqueous alkaline wash procedure, a crystallization procedure or a complexation procedure (for instance, with an amine compound, which results in an insoluble complex, which can be then isolated by centrifugation or filtration, CBD being released from the complex by combination with a solvent and acidification with HCl). It is also proposed, additionally to the first reaction mixture already comprising cannabidiol, to form a second reaction mixture by exposing the first reaction mixture to a terpene-containing component (for example, orange terpenes) to form a second reaction mixture; the subsequent CBD isolation can comprise separating an aqueous phase formed upon exposure of the second reaction mixture to the aqueous solvent from an organic phase or performing a complexation procedure or a crystallization procedure. The purity of the isolated CBD obtained after performing all steps is not clearly mentioned; the obtained CBD is said to be substantially pure, but, as defined in US2022144737A1, CBD can be substantially pure if it comprises less than 50% of an impurity.

In United States patent application US2022204431A1, in turn, it is disclosed a method for preparing cannabidiol and analogues thereof which is said to be simple in process, suitable for industrialization and which takes places in mild reaction conditions. The starting material are: terpenes of general formulas (II) or (VI) - which formulas encompass menthadienol and isopiperitenol when R₁ or R₄, respectively, are H -, and a phenolic compound of general formula (III) which encompasses olivetol when R₂ is H and R₃ is C₅H₁₁. The reaction can take place in a solvent, which can be an alkane, aromatic hydrocarbon, halogenated hydrocarbon, esters, ether and polar aprotic solvent, dichloromethane, 1,2-dichloroethane and chloroform being the possible examples of halogenated hydrocarbons explicitly mentioned. The compounds of formula (II) or (VI) and the compound of formula (III) are reacted in the presence of a catalyst M which catalyst, when R₁ or R₄ are different from H, may be selected from the group of Lewis acids, protic acids, acid anhydrides, silylesters and silanes; as examples of Lewis acids it is possible to find mentioned, among others, transition metal halides (among which titanium tetrachloride, zinc chloride and zinc bromide are the only mentioned examples).

However, when R₁. or R₄ are independently H (menthadienol or isopiperitenol, respectively), the catalyst may be selected from a more reduced group consisting of acid anhydrides, silylesters and silanes, not mentioning other possible options. No transition metal halide is used as catalyst in any of the Examples of US2022204431A1.

Even if R₃ is C₅H₁₁ in the starting compound of formula (III), when R₂ is different from H in said compound, in order to obtain cannabidiol it is necessary to perform a step, additional to the reaction of the compound of formula (II) or (VI) with the compound of formula (III), to remove R₂; in fact, most Examples of US2022204431A1 include that additional step. There is only one Example in said US application where the compound of formula (III) is olivetol (R₂ = H and R₃ = C₅H₁₁) and the compound of formula (II) is menthadienol (R₁ equal to H), an Example where such additional step is not necessary, but the whole process implies dissolving olivetol in dichloromethane/toluene added with anhydrous magnesium sulfate, slowly dropping the catalyst (trifluoromethanesulfonic anhydride in toluene), dropwise addition of menthadienol, addition of saturated sodium bicarbonate to quench the reaction, addition of water for layering, concentration of the organic phase to a small volume, addition of cyclohexane, methanol and a 10% KOH aqueous solution and layering, adjusting the organic phase to pH=6-7 with saturated tartaric acid aqueous solution, concentration to dryness, addition of n-heptane to the obtained light brown oil, filtering and drying of the precipitate, dissolving the obtained light white solid in n-heptane and stirring at -20°C and filtering and drying the precipitated solids. The NMR spectrum showed that the produced compound was cannabidiol, that was obtained with a total yield of 39.7%.

Other publications disclose methods of synthesis of not naturally-occurring cannabinoids. For instance, Chinese patent application CN113372196A discloses a method for synthesizing 8,9-dihydrocannabidiol, a compound with a structure similar to that of CBD but lacking a double bond in the exocyclic isopropyl group. The starting compounds are olivetol and α-phellandrene, which is a terpene with a certain structural similarity to menthadienol and isopiperitenol, but lacking -OH groups, having a saturated (lacking double bonds) isopropyl group and with two double bonds in the 6-membered ring. The reaction takes place in an organic solvent under the action of a Lewis acid catalyst and the method includes filtering of the reaction mixture when the yield of 8,9-dihydrocannabidiol does not increase any more, washing with saturated saline, solvent removal by distillation under reduced pressure, and purification of the crude product by silica gel column chromatography and elution with petroleum ether and ethyl acetate, followed by TLC elution. When the organic solvent is toluene and the catalyst is ferric chloride, the yield of 8,9-dihydrocannabidiol is 90%. However, other solvents give rise to lower yields, xylene and xylene/toluene being the only ones which give rise to yields higher of 50%, a yield of 49% being obtained with 1,2-dichloroethane. It is also shown that the particular catalyst used is very important, obtaining a yield of 90% only with ferric chloride, while the yield decreases to 53-54% with boron trifluoride monohydrate, boron trifluoride diethyl etherate and aluminium trichloride and is of only 50% with ferric chloride hexahydrate. Other reaction conditions, such as the catalyst amount, substrate concentration and reaction time, are also shown to be important. No method of obtaining CBD from 8,9-dihydrocannabidiol can be found in the document.

As can be seen, the main problems that are being encountered when designing different synthesis methods for CBD, until now, these can be summarized as: relatively low yield, simultaneous high THC content in the final product, presence of unwanted by-products such as abn-CBD and bis-CBD and/or the need for several steps to obtain the CBD, making it difficult to implement on an industrial scale.

It would be interesting to have a method that would make it possible to synthesize CBD with a high yield, a low abn-CBD and bis-CBD content and with a simple approach, to facilitate implementation at the industrial level. Preferably, the method should also make it possible to obtain CBD without high percentages of THC or, even, since THC itself is also a compound of interest, to be able to modify the percentages of CBD and THC present in the final product by modifying a simple adaptation factor within the synthesis method.

The present invention offers a solution to this problem.

### Summary of the invention

The invention relates to a synthesis method for a cannabinoid compound that is selected from cannabidiol (CBD) and Δ⁹-tetrahydrocannabinol (THC), comprising the steps of:
a) dissolving olivetol in dichloromethane (DCM) with stirring;
b) adding a Lewis acid as a catalyst;
c) keeping the suspension resulting from step b) under reflux for at least 20 minutes;
d) bringing the suspension obtained in c) to room temperature;
e) adding a solution of a terpene in dichloromethane dropwise and with stirring;
f) stirring for an additional 10 to 90 minutes after finishing adding the solution from e);
g) stopping the reaction;
h) purifying the synthesized cannabinoid;
wherein the catalyst added in phase b) is FeCl₃.6H₂O.

In a preferred embodiment of the invention, the terpene that is added in step e) is selected from (+)-p-mentha-2,8-dien-1-ol, (-)-p-mentha-2,8-dien-1-ol and isopiperitenol.

In another preferred embodiment, compatible with the above, the ratio of equivalents of the olivetol : terpene compounds is 1 : 1 and FeCl₃.6H₂O is between 0.05-0.6 eq.

In another preferred embodiment, compatible with the above, the suspension resulting from step b) is kept under reflux for 20-45 minutes.

In yet another preferred embodiment, compatible with any of the above, the terpene that is added in step e) is at a concentration of 0.015-0.0210 mmol/ml of DCM.

In yet another preferred embodiment, also compatible with any of the above, the reaction is stopped by adding an alkaline aqueous solution. The use of NHCO₃ is particularly preferred. When the reaction is stopped with an aqueous solution, it is preferred that the method includes a step in which the organic phase is separated and another step after it in which the organic phase is washed and, prior to the purification phase, dried.

In another preferred embodiment, also compatible with any of the above, the synthesized cannabinoid is purified by chromatography. Flash column chromatography on silica is particularly preferred.

In particularly preferred embodiments of the invention, compatible with any of the above, although some of them represent particular cases of the previous general embodiments or combinations of limitations of some of the previous possible preferred embodiments:
i) the cannabinoid to be synthesized is cannabidiol and the stirring time of step f) is selected in the range of 10 to 25 minutes, the value of 20 minutes being particularly preferred,
ii) the cannabinoid to be synthesized is Δ⁹-tetrahydrocannabinol (THC) and the stirring time of step f) is selected between 25 and 90 minutes, the value of 90 minutes being particularly preferred.

It is particularly preferred that:
i. when the cannabinoid to be synthesized is cannabidiol and the selected terpene is (+)-p-mentha-2,8-dien-1-ol, the time range of step f) is selected from the range of 15-25 minutes,
ii. when the cannabinoid to be synthesized is cannabidiol and the selected terpene is isopiperitenol, the time range of step f) is selected from the range of 10-20 minutes,
iii. when the cannabinoid to be synthesized is Δ⁹-tetrahydrocannabinol (THC) and the selected terpene is (+)-p-mentha-2,8-dien-1-ol, the time range of step f) is selected from the range of 25-90 minutes,
iv. when the cannabinoid to be synthesized is Δ⁹-tetrahydrocannabinol (THC) and the selected terpene is isopiperitenol, the time range of step f) is selected from the range of 20-90 minutes.

### Brief Description of the Figures

Fig. 1. Main cannabinoids of *Cannabis sativa.*
Fig. 2. Biosynthesis scheme of the main cannabinoids.
Fig. 3. First CBD syntheses due to Petrzilka (panel A), Rickards (panel B) and Mechoulam (panel C).
Fig. 4. General synthesis of CBD by direct coupling with (+)-p-mentha-2,8-dien-1-ol
Fig. 5. CBD synthesis by direct coupling with limonene derivatives.
Fig. 6. Nuclear magnetic resonance spectrograms obtained from (-)-trans-CBD. Panel A, 1H-NMR; panel B, 13C-NMR.
Fig. 7. Nuclear magnetic resonance spectrograms obtained from (-)-trans-Δ⁹-THC. Panel A, 1H-NMR; panel B, 13C-NMR.
Fig. 8, panels A, B, C and D. 4 chromatograms are shown in which it can be seen how Abn-CBD disappears in the reaction catalyzed by FeCl₃·6H₂O (retention time: 8.89 min) as a by-product, The peak with a retention time of 9.04 corresponds to CBD. In the first three chromatograms, Yb(OTf)₃ (panel A), camphorsulfonic acid (CSA) (panel B) and para-toluenesulfonic acid (panel C) are used as catalysts, respectively; in the last one (panel D), FeCl₃·6H₂O is used.
Fig. 9. Nuclear magnetic resonance (NMR) spectrograms of Abn-CBD (upper panel, A) and bis-CBD (lower panel, B).

### Detailed description of the invention

As mentioned, the present invention relates to a method for the synthesis of cannabidiol (CBD) that allows obtaining Δ⁹-tetrahydrocannabinol (THC), simply by varying the time during which a step of the reaction takes place, the stirring step that takes place once all the reagents have been added and varying the number of equivalents of FeCl₃.6H₂O. Each one of said compounds is obtained with a high yield under its proper reaction conditions, with a low content of unwanted by-products such as abn-CBD and bis-CBD.

Furthermore, the method can be carried out with different terpenes; specifically, this specification shows examples of use of both the terpene (+)-p-mentha-2,8-dien-1-ol (25) and another less expensive terpene, derived from limonene, isopiperitenol.

Specifically, the main advantages of the synthesis method of the present invention are the following:
- High CBD yield (95%) with minimal formation of by-products such as abn-CBD or bis-CBD at trace level
- Use of a catalyst such as FeCl₃.6H₂O that it is inexpensive and non-toxic
- Simple method of preparation and elaboration of the synthesis process
- Mild reaction conditions

The method of the present invention, when considered in its variant targeting CBD synthesis in particular, can be considered as a variation of the CBD synthesis methods by direct coupling discussed in the "Background of the invention" section, particularly those generalized in Schemes 4 or 5. In fact, the process could be simplified by means of a scheme of the following type: 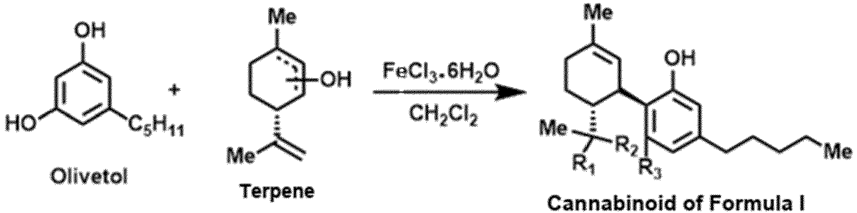 wherein, in the terpene formula, the dashed line indicates that the double bond can be in one of two possible locations and the OH can also vary its location between the positions encompassed by the dashed line. The terpene is preferably, according to its IUPAC nomenclature, 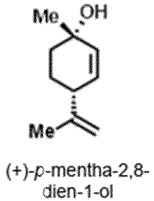 (1*S*,4*R*)-1-methyl-4-prop-1-en-2-ylcyclohex-2-en-1-ol, a compound sometimes referred to simply as (+)-menthadienol,
or 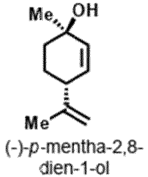 according to its IUPAC nomenclature, is (1*R*,4*R*)-1-methyl-4-prop-1-en-2-ylcyclohex-2-en-1-ol, a compound sometimes referred to simply as (-)-menthadienol,
or 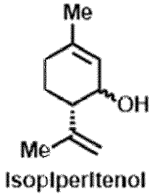 a limonene derivative which, according to its IUPAC nomenclature, is *(2S,R, 6R)-3-*methyl-6-prop-1-en-2-ylcyclohex-2-en-1-ol. This compound (understanding as such the two possible isomers on the carbon where the -OH group is) has the advantage that it is less expensive than (+)-*p*-mentha-2,8-dien-1-ol. Other terpenes that have previously been used for CBD synthesis and/or that present a methylcyclohexenol substituted with a prop-1-en-2-yl group in *para* with respect to the methyl substituent can be also used

As for the cannabinoid of Formula I, it would be a cannabinoid in which:
when R1 is H, R2 is =CH₂ and R3 is OH (CBD: cannabidiol), or
when R1 is Me, R2 and R3 together form a -O- group, in such a way that they give rise to a cycle in which the -O- group connects the carbon to which R3 is attached with the carbon to which R2 (Δ⁹-THC: Δ⁹-tetrahydrocannabinol) is attached

However, the method of the present invention presents an important variation with respect to any of the previously disclosed CBD synthesis processes by direct coupling of olivetol with terpenes, in particular with those specified in Tables 1 or 2, or with the previous method of the Mechoulam group (Baek *et al.,* 1985) or with the methods described in patent applications US2022144737A1 or US2022204431A1: the use of a catalyst that is not used in any of them, FeCl₃.6H₂O, which, as mentioned before, is inexpensive and non-toxic.

The combination of reaction conditions of the method of the present invention is in turn a difference with respect to previously known CBD synthesis methods by direct coupling, both those that are carried out with (+)-p-mentha-2,8-dien-1-ol and those performed with limonene derivatives: use of FeCl₃.6H₂O as a catalyst, dichloromethane (CH₂Cl₂, often abbreviated as DCM) as a solvent, and reaction at room temperature after a previous step in which olivetol and the catalyst FeCl₃.6H₂O, already dissolved in dichloromethane, have been kept under reflux of the solvent and under strong stirring.

These differences give rise, as indicated above, to a reaction in which CBD is obtained with a high yield and little formation of abn-CBD or bis-CBD by-products. The reaction conditions with which it is achieved are simple and easy to implement at an industrial level.

The use of such reaction conditions, particularly the use of FeCl₃.6H₂O as a catalyst, and their effect, are not obvious to be conceived from the state of the art, specifically not from the teachings of any of the documents mentioned in the section of "Background of the Invention" and particularly not from the teachings of patent applications US2022144737A1 and US2022204431A1, where such compound is not mentioned as a possible catalyst to be used to produce CBD, even though many other Lewis acids are mentioned as possible options. In the particular case of US2022204431A1, it is remarkable that its teachings, even, seem to teach away from the use of any transition metal halide as catalyst when the reaction takes place with menthadienol or isopiperitenol as starting compounds, because they are not mentioned in the list of possible catalyst for such case, while such catalysts are mentioned as possible options (but not used in any of the US2022204431A1 Examples) when other terpenes are used as starting compounds. In US2022144737A1, in turn, the only catalyst used in the Examples is not even a halide, but Zn(OTf)₃, and dichloromethane is not one of the solvents used in the exemplary reactions to form crude cannabidiol.

However, the use of FeCl₃.6H₂O as catalyst, also favored by the use of dichloromethane as solvent, gives rise to a process to obtain purified CBD which is simpler than that of US2022144737A1, where steps such as complexation with amines and/or crystallization are provided (and used in the Examples) to facilitate CBD purification, and where even a second reaction with terpenes is proposed, in order to obtain CBD where the main impurities are GRAS ("generally recognized as safe"). The purity of the CBD finally obtained is not specifically mentioned for each exemplified process, being only said, as a general conclusion, that it ranges from greater than 50% to less than 100%, being enough (for the purposes of the invention of said patent application) that CBD comprises less than 50% of impurities to define CBD as "substantially pure".

With regard to US2022204431A1, the yields obtained with the present invention are clearly higher since, in the only Example of US2022204431A1 where olivetol is reacted with one of the preferred terpenes of the present invention (menthadienol), thus allowing to obtain CBD directly without additional reaction steps, a total yield of 39.7% is reported.

The use of FeCl₃.6H₂O and dichloromethane as solvent and the advantages obtained from that could not have been expected, either, having knowledge of documents disclosing the synthesis of CBD analogues such as 8,9-dihydrocannabidiol using as starting compounds olivetol and a terpene which is specifically α-phellandrene, such as patent application CN113372196A. In said application, the possible use of FeCl₃.6H₂O as catalyst is mentioned. However, it is remarkable that the Example of CN113372196A where the effect of different catalysts is compared shows that FeCl₃.6H₂O gives rise to a yield of only 50%, which is lower that the yield of some others of the assayed catalysts, and particularly much lower than that obtained with FeCl₃, what probably would have led one skilled in the art to discard FeCl₃.6H₂O as a catalyst to be assayed for CBD synthesis or, at least, would have provoked that that person did not try to perform assays with such catalyst for obtaining CBD having reasonable expectations of achieving advantageous effects such as those of the present invention. Besides, the solvent that gives rise to better results in CN113372196A is toluene, not dichloromethane, which is not even mentioned in said application as a possible solvent.

And, what is more, some meaningful structural differences between α-phellandrene and the preferred terpenes of the present invention, such as (±)-mentha-2,8-dien-1-ol and isopiperitenol, would have led to one skilled in the art to discard the teachings of CN113372196A as good starting point to design a new process for obtaining CBD with the advantages achieved with the method of the present invention, because that person would have had serious doubts about the reproducibility of the results obtained with the method of CN113372196A in a process for CBD synthesis using as a starting compound any compound with the structures of the terpenes usable for the purposes of the present invention, as defined above, where a double bond is present in the isopropyl group (that is, an exocyclic double bond is present in the terpenic unit) and only a double bond is present in the 6-membered ring. The presence or absence of the exocyclic double bond gives rise to a completely different chemical behaviour both of the starting terpene and the final product in the presence of the Lewis acid which is being used as catalyst in the reaction. Double bonds are more susceptible to chemical reactions and increase molecular reactivity in several senses, for instance in addition reactions; besides, double bonds affect compound stability. It is demonstrated that the yield of a product without a specific double bond is always higher than the yield of those products having such double bond. Consequently, synthesizing natural cannabinoids as CBD means a challenge with regard to 8,9-dihydro-analogues, as the compound synthesized in CN113372196A, due to the presence of the exocyclic double bond, and that implies that the teachings of CN113372196A1, where FeCl₃ is used as catalyst, cannot be extrapolated directly to CBD synthesis, because the chemical behaviour of, for instance, mentha-2,8-dien-1-ol or isopiperitenol cannot be expected to be the same. In fact, if the 8,9-dihydro-compound synthesized according to the method of CN113372196A1 is allowed to remain in the acid medium where the reaction has occurred, it evolves to give rise to different compounds. Thus, it could not be expected that the results obtained in CN113372196A with the terpene α-phellandrene and FeCl₃ could be extrapolated to other terpenes used as starting compounds and having an exocyclic double bond like the one of mentha-2,8-dien-1-ol or isopiperitenol; indeed, one skilled in the art would have expected, to the contrary, yields more similar to those obtained with most of the Lewis acids assays in CN113372196A1, which are in the range of 35-55%. Therefore, one skilled in the art would not have combined the teachings of CN113372196A with those of US2022144737A1 and/or US2022204431A1 in order to design a method like the one of the present invention, because that person would have had serious doubts about the results obtained due to the structural differences of the terpenes to be used as starting compounds. Moreover, as explained above, it would not have arrived at conceiving the method of the present invention, because FeCl₃.H₂O is used as catalyst in the present invention and it does not give rise to high yields in the assays of CN113372196A.

Furthermore, depending on the reaction time, the method of the invention allows obtaining either CBD or Δ⁹-THC. The latter would be the product obtained by prolonging the reaction time.

It can be said that the time that the reaction is allowed to pass from the time the terpene is added until the reaction stops can range from 10 to 90 minutes, depending on the compound to be obtained and the preferred yield thereof. There are also specific preference ranges depending on whether (+)-p-mentha-2,8-dien-1-ol or isopiperitenol is used. This is explained below in more detail.

Using (+)-p-mentha-2,8-dien-1-ol as the starting terpene and considering as t = 0 min the time at which the addition of the terpene to the olivetol and FeCl₃·6H₂O solution ends, the optimal time range (in which only bis-CBD is obtained as a by-product) is 15-25 min. If the reaction is stopped within 0-15 min, a mixture of CBD, starting reagents and some bis-CBD are obtained. After 25 min, THC begins to be generated in the reaction. The same result is obtained using (-)-p-mentha-2,8-dien-1-ol as the starting terpene.

Using isopiperitenol as the starting terpene and considering as t = 0 min the time at which the addition of the terpene to the olivetol and FeCl₃·6H₂O solution ends, the optimal time range (in which only bis-CBD is obtained as a by-product) is 10-20 min. If the reaction is stopped within 0-10 min, a mixture of CBD, starting reagents and some bis-CBD are obtained. After 20 min, THC begins to be generated in the reaction.

In that sense, when CBD is to be synthesized, a possible preferred value for the time the reaction is allowed to proceed from the completion of terpene addition can be considered to be 20 minutes. Specifically, when the synthesis is performed with (+)-p-mentha-2,8-dien-1-ol, the preferred stirring time range from completion of terpene addition is 15-25 minutes; when the synthesis is performed with isopiperitenol, the preferred stirring time range from completion of terpene addition is 10-20 minutes.

On the other hand, the generation of bis-CBD as a by-product depends on: the rate of addition and the concentration. In conclusion, for a 0.02 M concentration of terpene in the additions the time of which is less than 60 s/g, a greater amount of bis-CBD is observed, whereas in additions the time of which is greater than or equal to 60 s/g of terpene, the generation of the by-product decreases. Terpene concentration ranges that have been tested range from 0.003 M to 0.02 M.

On the other hand, as for TCH, using (+)-p-mentha-2,8-dien-1-ol as the starting terpene and considering as t = 0 min the time at which the addition of the terpene to the olivetol and FeCl₃·6H₂O solution ends, the time needed to obtain THC is between 25-90 min. Between 25 min and 90 min if the reaction is not complete. After 90 min, the reaction ends without advancing to other by-products.

Using isopiperitenol as the starting terpene and considering as t = 0 min the time at which the addition of the terpene to the olivetol and FeCl₃·6H₂O solution ends, the optimal time range for THC synthesis (in which only bis-CBD is obtained as a by-product) is 20-90 min (this time range is the time it takes for the reaction to complete). If the reaction stops within 0-15 min, a mixture of THC, CBD, starting reagents and some bis-CBD are obtained. After 90 min, the reaction does not progress.

In that sense, when CBD is to be synthesized, a possible preferred value for the time the reaction is allowed to proceed from the completion of terpene addition can be considered to be 20 minutes. Specifically, when the synthesis is performed with (+)-p-mentha-2,8-dien-1-ol, the preferred stirring time range from completion of terpene addition is 15-25 minutes; when the synthesis is performed with isopiperitenol, the preferred stirring time range from completion of terpene addition is 10-20 minutes

In that sense, when THC is to be synthesized, a possible preferred value for the time the reaction is allowed to proceed from the completion of terpene addition can be considered to be 90 minutes. Specifically, when the synthesis is performed with (+)-p-mentha-2,8-dien-1-ol, the preferred stirring time range from completion of terpene addition is 25-90 minutes; when the synthesis is performed with isopiperitenol, the preferred stirring time range from completion of terpene addition is 25-90 minutes.

The presence of bis-CBD and abn-CBD was detected by both thin-layer chromatography and gas chromatography coupled to mass spectrometry (GC-MS), as described in Example 5 below.

With respect to the ratio of compounds to be used in the reaction, the preferred ratio of equivalents of the olivetol : FeCl3.6H₂O : terpene compounds is 1 : 0.05-0.6 :1. In particular, to synthesize CBD the preferred ratio of equivalents is 1 : 0.05-0.1 : 1, while to synthesize THC the preferred ratio of equivalents is 1: 0.5-0.6 : 1.

As discussed, the synthesis method starts from a terpene solution in DCM (preferably, 100 ml of DCM per 1 mmol of terpene), intensely stirred. Intense stirring means values close to 650-1000 rpm. FeCl₃.6H₂O is added to this solution and the resulting suspension is subjected to reflux. This results in a color change from pale yellow to intense yellow or orange being observed. 20 minutes at reflux may be enough, although it may take longer, for example up to 45 minutes.

After the mentioned time range at reflux, the suspension is allowed to reach room temperature (t^{a}). Then a solution of the selected terpene in DCM is added dropwise. A possible ratio of the terpene to DCM would be 0.015-0.021 mmol/ml DCM; when the terpene is (+)-p-mentha-2,8-dien-1-ol, the preferred ratio is close to 0.0200 mmol/ml DCM (for example, 6.00-6.11 mmol in 300 ml of DCM, which corresponds to approximately 1 equivalent in 300 ml of DCM, as in Examples 1 and 3 below), while when the terpene is isopiperitenol, the preferred ratio is closer to 0.0165 mmol/mL DCM (for example, 0.33 mmol in 20 ml of DCM, which corresponds to 1 equivalent in 20 ml of DCM, as in Examples 2 and 4 below). To add a total of 20 to 300 ml of the solution of terpene in DCM, between 5 and 20 minutes is a suitable time range.

When the dropwise addition of the terpene is complete, the reaction is allowed to proceed, under stirring at 400-600 rpm, for a time that is selected according to the cannabinoid to be synthesized and the selected terpene, also taking into account avoiding values in which by-products such as bis-CBD or abn-CBD are mainly obtained, as described above.

After the chosen time has elapsed, the reaction is stopped. The reaction can be stopped by various methods, known to those skilled in the art. One possibility is to use an aqueous solution the pH of which is equal to or greater than 7, more preferably an alkaline aqueous solution (pH greater than 7). Saturated NaHCO₃ is preferred. And to facilitate further processing, it can be concentrated under reduced pressure, for example in rotary evaporator, until having half the volume of DCM. After that, especially if the reaction has been stopped with an alkaline aqueous solution, it is washed, for example sequentially with NaHCO₃, water and brine, and dried, for example on anhydrous MgSO₄ and concentrated under reduced pressure until obtaining a crude product, pending purification, as can be seen in Synthesis Examples 1 to 4 below.

Purification can occur by any method known to those skilled in the art, such as column chromatography or crystallization, the latter method being valid for CBD. The use of chromatography is preferred, for example flash column chromatography on silica. With it, CBD or THC is obtained, depending on the chosen conditions, with a high yield and purity.

Below are examples of CBD and THC synthesis, which are particularly preferred specific embodiments of the present invention. An example of identifying by-products depending on the reaction conditions is also described.

### Examples

### - Example 1. CBD synthesis using (+)-p-mentha-2,8-dien-1-ol

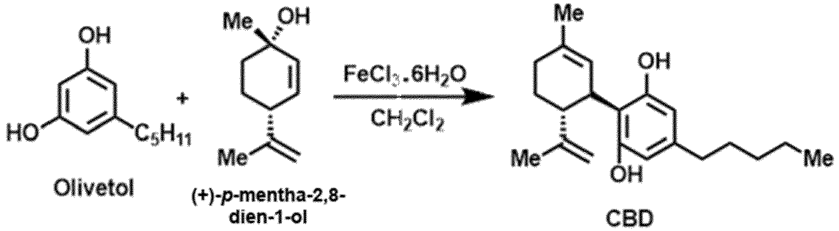

Method : FeCl₃·6H₂O (0.165 g, 0.611 mmol, 0.1 equiv) were added to a strongly stirred solution of olivetol (1.101 g, 6.00 mmol, 1.0 equiv) in DCM (600 mL) and the resulting solution was brought to reflux (a color change from pale yellow to deep yellow or orange was observed). After 20 min., the suspension was allowed to reach room temperature and a solution of (+)-p-mentha-2,8-dien-1-ol (0.915 g, 6.00 mmol, 1.0 equiv) in DCM (300 mL) was added dropwise for 20 min. When the addition was complete, the reaction was kept under stirring for an additional 10 minutes. After that time, the reaction was stopped with saturated NaHCO₃ (20 mL) and concentrated to half the volume of DCM. The organic phase was separated and washed sequentially with NaHCO₃ (100 mL), water (2 x 100 ml), brine (100 ml), dried on anhydrous MgSO₄ and concentrated under reduced pressure until obtaining a crude product, which was purified by flash column chromatography on silica to obtain CBD (1.83 g, 95%) as a white solid.

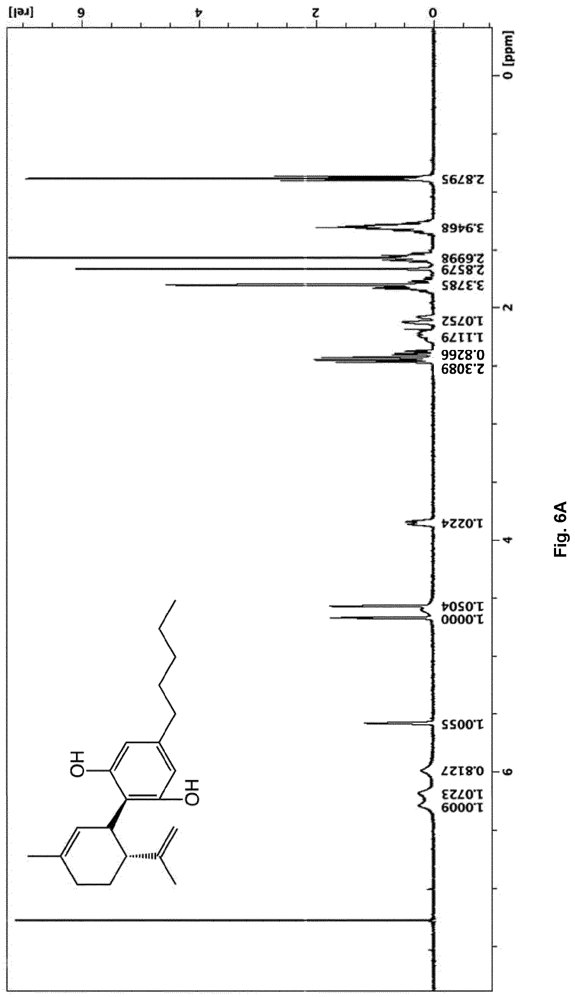

Spectroscopic data: 1H-NMR (500 MHz, CDCl3): ∂ 6.34-6.10 (m, 2H), 5.97 (brs, 1H), 5.57 (s, 1H), 4.66 (s, 1H), 4.06 (brs, 1H), 4.56 (s, 1H), 3.89-3.80 (m, 1H), 2.44 (t, 2H, 7.80 Hz), 2.41-2.36 (m, 1H), 2.29-2.18 (m, 1H), 2.13-2.06 (m, 1H), 1.87-1.83 (m, 1H), 1.80 (s, 3H), 1.65 (s, 3H) 1.56 (s, 3H), 1.36-1.27 (m, 4H), 0.88 (t, 3H, 7.02 Hz). The spectrogram can be seen in Fig. 6A.

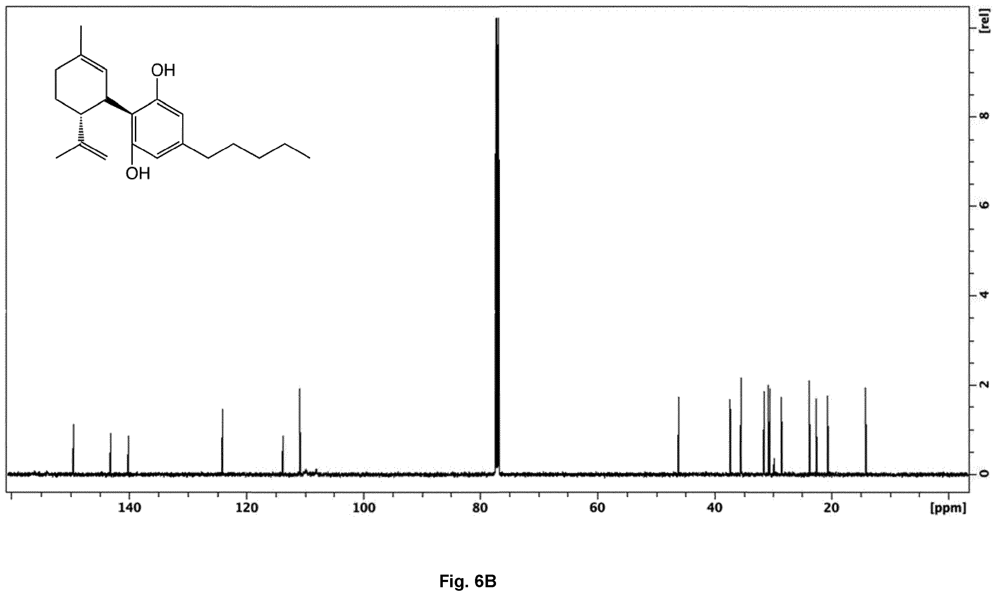

¹³C-NMR (125.7 MHz, CDCl3): ∂ 149.42, 143.06, 140.07, 124.11, 113.74, 110.83, 109.86, 108.00, 46.14, 37.40, 35.48, 31.49, 30.64, 30.41, 28.41, 23.69, 22.54, 20.56, 14.04. The spectrogram can be seen in Fig. 6B.

### - Example 2. CBD synthesis from isopiperitenol

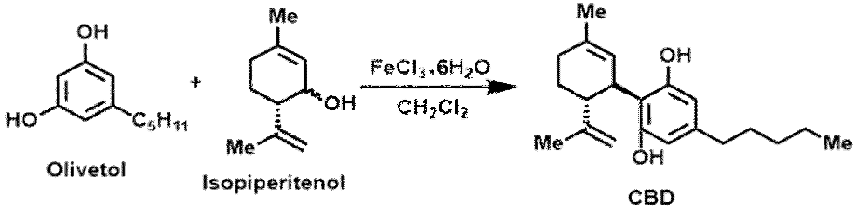

### Method:

Isopiperitenol was prepared from R-limonene following the method described by Dethe *et al.,* 2015. Briefly, it consists of an initial treatment with CrO₃ in tert-butanol and, subsequently, a treatment with NaBH₄ and CeCl₃. Specifically, 24 g (240.0 mmol, 4 eq.) of chromium(VI) oxide were added in small portions on 2-methyl-2-propanol (60 ml) and allowed to stir for 15 min. Next, the solution was extracted with toluene (160 ml), which was subsequently dried on anhydrous magnesium sulfate and concentrated to half its volume (80 mL). 9.5 ml (58.7 mmol, 1 eq.) of limonene were added to this solution and it was brought to 80 °C for 2 h. After this time, it was filtered using celite as a filter aid, washing three times with diethyl ether, to obtain a solution that was concentrated under reduced pressure to give a crude product that was purified by flash column chromatography, thus obtaining isopiperitenone. The ketone obtained in the previous step was dissolved in anhydrous methanol, 0.349 g (0.936 mmol, 0.5 eq.) of cerium trichloride heptahydrate were added and it was kept under stirring until the mixture was homogeneous. Next, the solution was brought to 0 °C to add 0.0378 g (2.245 mmol, 1.2 eq.) of sodium borohydride in portions and the mixture was stirred for 30 min. After this time, the reaction was quenched with water, extracted three times with dichloromethane and the organic phase was washed with brine, dried on anhydrous magnesium sulfate and concentrated under reduced pressure to obtain a crude product which was purified by flash column chromatography to obtain isopiperitenol.

As for the synthesis of CBD itself, the following method was used: FeCl₃·6H₂O (4.5 mg, 0.017 mmol, 0.05 equiv) were added to a strongly stirred solution of olivetol (60 mg, 0.33 mmol, 1.0 equiv) in DCM (30 mL) and the resulting solution was brought to reflux (a color change from pale yellow to deep yellow or orange was observed). After 20 min., the suspension was allowed to reach room temperature and a solution of isopiperitenol (50 mg, 0.33 mmol, 1.0 equiv) in DCM (20 mL) was added dropwise for 5 min. When the addition was complete, the reaction was kept under stirring for an additional 10 minutes. After that time, the reaction was stopped with saturated NaHCO₃ (20 mL) and concentrated to half the volume of DCM. The organic phase was separated and washed sequentially with NaHCO₃ (20 ml), water (2 x 20 ml), brine (50 ml), dried on anhydrous MgSO₄ and concentrated under reduced pressure until obtaining a crude product, which was purified by flash column chromatography on silica to obtain CBD (95 mg, 92 %) as a white solid

Spectroscopic data: ¹H-NMR (500 MHz, CDCl3): ∂ 6.34-6.10 (m, 2H), 5.97 (brs, 1H), 5.57 (s, 1H), 4.66 (s, 1H), 4.06 (brs, 1H), 4.56 (s, 1H), 3.89-3.80 (m, 1H), 2.44 (t, 2H, 7.80 Hz), 2.41-2.36 (m, 1H), 2.29-2.18 (m, 1H), 2.13-2.06 (m, 1H), 1.87-1.83 (m, 1H), 1.80 (s, 3H), 1.65 (s, 3H) 1.56 (s, 3H), 1.36-1.27 (m, 4H), 0.88 (t, 3H, 7.02 Hz).

¹³C-NMR (125.7 MH, CDCl3): ∂ 149.42, 143.06, 140.07, 124.11, 113.74, 110.83, 109.86, 108.00, 46.14, 37.40, 35.48, 31.49, 30.64, 30.41, 28.41, 23.69, 22.54, 20.56, 14.04.

### - Example 3. Δ⁹-THC synthesis from p-mentha-2,8-dien-1-ol

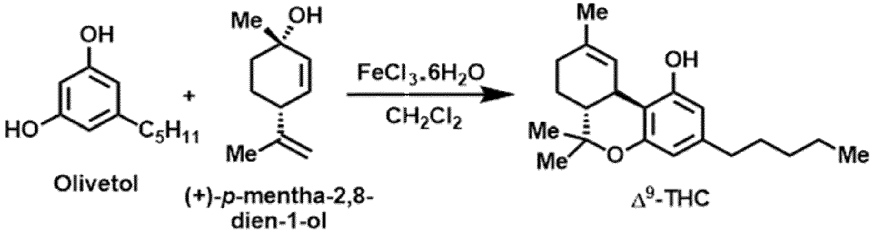

Method: FeCl₃·6H₂O (0.82 g, 3.06 mmol, 0.5 equiv) were added to a strongly stirred solution of olivetol (1.08 g, 6.11 mmol, 1.0 equiv) in DCM (600 mL) and the resulting solution was brought to reflux (a color change from pale yellow to deep yellow or orange was observed). After 20 min., the suspension was allowed to reach room temperature and a solution of (+)-p-mentha-2,8-dien-1-ol (0.930 g, 6.11 mmol, 1.0 equiv) in DCM (300 mL) was added dropwise for 20 min. When the addition was complete, the reaction was kept under stirring for an additional 90 minutes. After that time, the reaction was stopped with saturated NaHCO₃ (20 mL) and concentrated to half the volume of DCM. The organic phase was separated and washed sequentially with NaHCO3 (100 ml), water (2 x 100 ml), brine (100 ml), dried on anhydrous MgSO4 and concentrated under reduced pressure until obtaining a crude product, which was purified by flash column chromatography on silica to obtain THC (1.75 g, 91%) as a yellow oil.

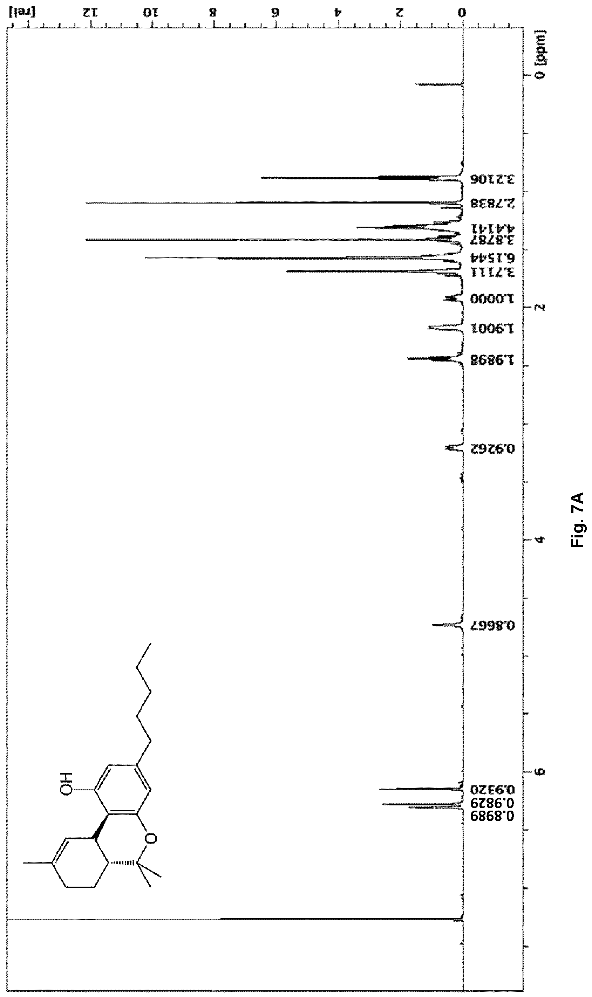

Spectroscopic data: ¹H-NMR (500 MHz, CDCl3): ∂ 6.29 (t, 1H, 1.49 Hz), 6.27 (d, 1H, 1.30 Hz), 6.14 (d, 1H, 1.30 Hz), 4.90 (s, 1H), 3.23-3.16 (m, 1H), 2.43 (td, 2H, 11.55 Hz, 3.58 Hz), 2.20-2.13 (m, 2H), 1.95-1.89 (m, 1H) 1.70-1.66 (s, 4H), 1.58-1.54 (m, 2H), 1.41 (m, 4H), 1.34-1.28 (m), 1.26 (s), 1.09 (s, 3H), 0.88 (t, 6.90 Hz). The spectrogram can be seen in Fig. 7A

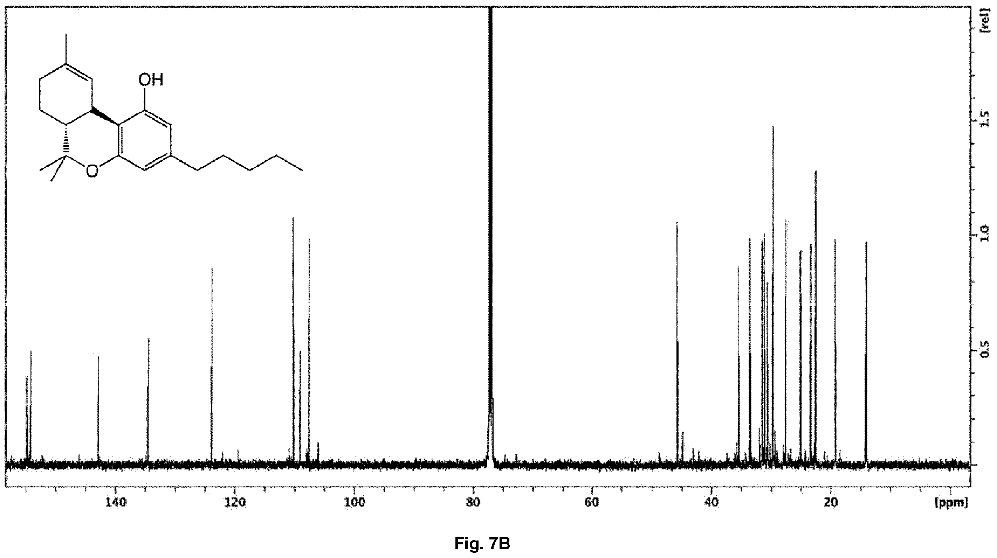

¹³C-NMR (125.7 MH, CDCl3): ∂ 154.79, 154.13, 142.84, 134.46, 123.70, 110.12, 109.02, 107.53, 45.80, 35.48, 35.57, 31.52, 31.17, 30.66, 29.71, 27.58, 25.02, 23.38, 22.56, 19.28, 14.02. The spectrogram can be seen in Fig. 7B.

### - Example 4. Δ⁹-THC synthesis from isopiperitenol

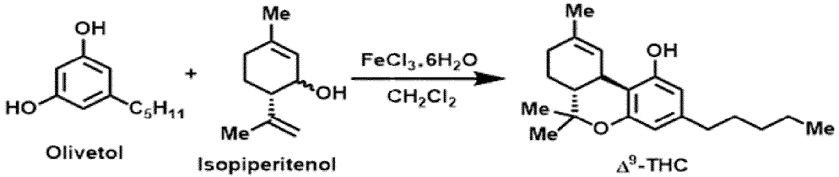

Method: FeCl₃·6H₂O (54.0 mg, 0.20 mmol, 0.6 equiv) were added to a strongly stirred solution of olivetol (60 mg, 0.33 mmol, 1.0 equiv) in DCM (30 mL) and the resulting solution was brought to reflux (a color change from pale yellow to deep yellow or orange was observed). After 20 min., the suspension was allowed to reach room temperature and a solution of isopiperitenol (50 mg, 0.33 mmol, 1.0 equiv) in DCM (20 mL) was added dropwise for 5 min. When the addition was complete, the reaction was kept under stirring for an additional 90 minutes. After that time, the reaction was stopped with saturated NaHCO₃ (20 mL) and concentrated to half the volume of DCM. The organic phase was separated and washed sequentially with NaHCO₃ (20 ml), water (2 x 20 ml), brine (20 ml), dried on anhydrous MgSO₄ and concentrated under reduced pressure until obtaining a crude product, which was purified by flash column chromatography on silica to obtain THC (97 mg, 94 %) as a yellow oil.

### - Example 5. Checking the generation of abn-CBD and bis-CBD

The tests described in Examples 1 to 4 were repeated, but varying the time during which the reaction is allowed to progress from when terpene addition is finished, as well as the type of acid added. Samples were taken at different times to check for the presence of abn-CBD and bis-CBD.

The presence of these substances was detected by both thin-layer chromatography and gas chromatography coupled to mass spectrometry (GC-MS).

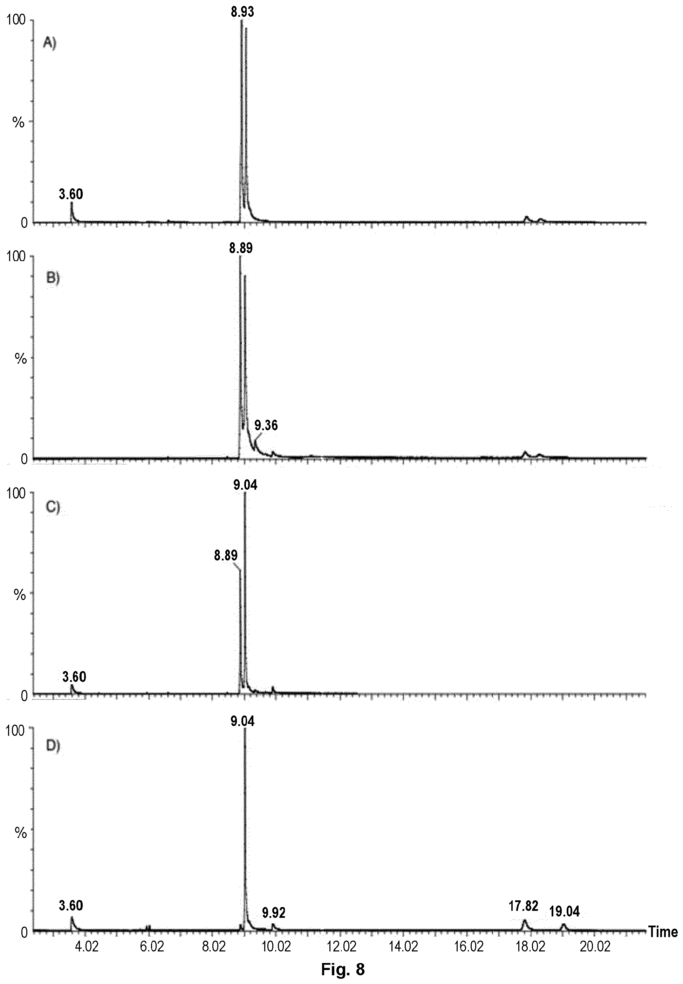

In Fig. 8, panels A, B, C and D, 4 chromatograms are shown in which it can be seen how in the reaction catalyzed by FeCl₃·6H₂O, Abn-CBD (with a retention time of 8.89 min) disappears as a by-product. The peak with a retention time of 9.04 corresponds to CBD. In the first three chromatograms, Yb(OTf)₃, camphorsulfonic acid (CSA) and para-toluenesulfonic acid are used as catalysts, respectively; whereas in the last one, FeCl₃·6H₂O is used. In that sense, it can be seen that the abn-CBD by-product was not formed at all by this method using FeCl₃.6H₂O as a catalyst. The spectra obtained show that, when other acids are used, the by-product is formed and also sometimes in very large quantities, as shown for example by the chromatograms of the reaction when Yb(OTf)₃, CSA or pTsOH (chromatograms A, B and C in Fig. 8) were used.

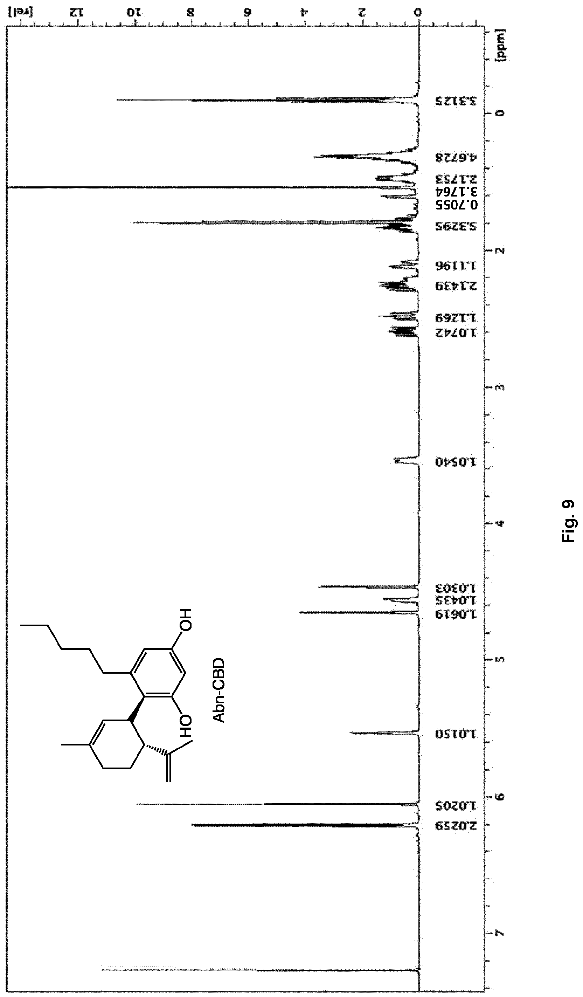

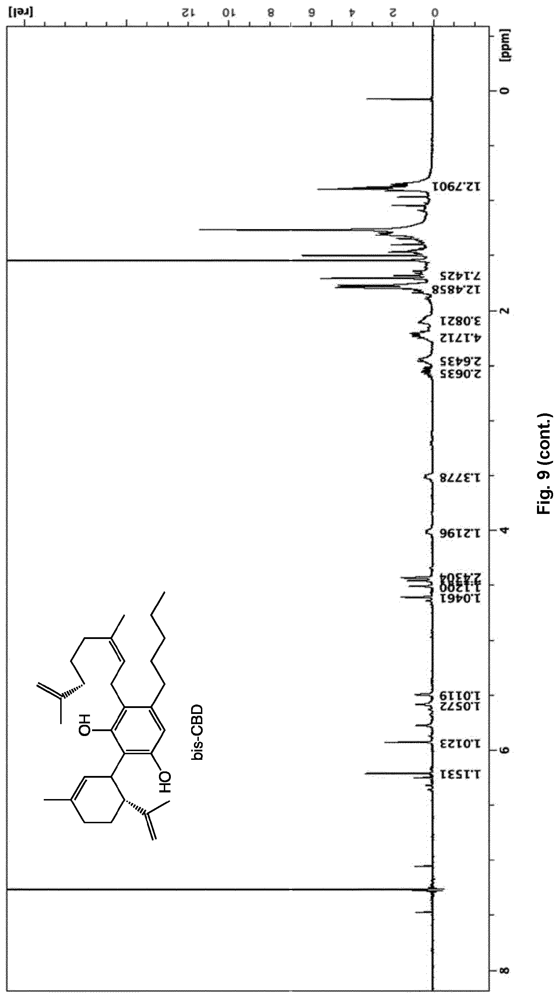

Furthermore, the purification of the reaction products and their subsequent study by means of nuclear magnetic resonance allowed confirming their structure and comparing it with those described in the literature: The results of the spectroscopic studies that confirmed that the monitored compounds were abn-CBD and bis-CBD are shown in Fig. 9.

### References

Anand R., S. Sharma, C. P. Singh, V. Gannedi, M. Kumar, S. V. Pratap, R. V. Prakash, V. R. Asrey, S. P. Pal. Process for the Synthesis of Cannabidiol and Intermediates Thereof. WO 2021181420 A1. 2021.
Anand R., P. S. Cham, V. Gannedi, S. Sharma, M. Kumar, B. Singh, R. A. Vishmakarna, P. P. Singh. Stereoselective Synthesis of Nonpsychotic Natural Cannabidiol and Its Unnatural/Terpenyl/Tail-Modified Analogues. J. Org. Chem. 2022, 87, 4489-44998.
Baek S.H., M. Srebnik, R. Mechoulam. Boron triflouride etherate on alimina-a modified Lewis acid reagent.: An improved synthesis of cannabidiol. Tetrahedron Lett., 1985, 26, 1083
Bailey S.J., R. R. Sapkota, A. E. Golliher, B. Dungan, M. Talipov, F. O. Holguin, W. A. Maio. Lewis-Acid-Mediated Union of Epoxy-Carvone Diastereoisomers with Anisole Derivatives: Mechanistic Insight and Application to the Synthesis of Non-Natural CBD Analogues. Org. Lett. 2018, 20, 4618-4621
Bencivenga M., M. Forster, P. Herrinton, P. Jass, S. Singh, T. Zahn. Synthetic Cannabidiol Compositions and Methods of Making the Same. WO 2019046806 A1. 2019.
Bloemendal V. R. L. J., B. Spierenburg, T. J. Boltje, J. C. M. van Hest, F. P. J. T. Rutjes. One-Flow Synthesis of Tetrahydrocannabinol and Cannabidiol Using Homo- and Heterogenous Lewis Acid. J. Flow Chem. 2021, 11, 99-105.
Burdick D.C., S. J. Collier, F. Jos, B. Biolatto, B. J. Paul, H. Meckler , M. A. Helle, A. J. Habershaw. Process for Production of Delta-9-Tetrahydrocannabinol. WO 2007041167 A2, 2007.
Burdick D.C., S. J. Collier, F. Jos, B. Biolatto; B. J. Paul, H. Meckler, M. A. Helle, A. J. Habershaw. Process for production of delta-9-tetrahydrocannabinol. U.S. Patent 2007093665 A, 2014
Cardillo B., L. Merlini, S. Servi. Alkylation of resorcinols with monterpenoid allylic alcohols in aqueous acid. Synthesis of new cannabinoid derivatives. Tetrahedron Lett. 1972, 13, 945-948
Chen J., B. Li, Z. Gu. Cannabidiol intermediate and preparation method and application thereof. CN109734591. 2018
Dethe D.H., R. D. Erande, S. Mahapatra, S. Das, V. Kumar B. Chem. Commun. 2015, 51, 2871-2873
Dialer L., D. Petrovic, U. Weigl. Process for the Production of Cannabidiol and Delta-9-Tetrahydrocannabinol. U.S. 20170008868 A1. 2017.
Dialer L., D. Petrovic, U. Weigl. Process for the Production of Cannabidiol and Delta-9-Tetrahydrocannabinol. WO 2017011210 A1. 2015.
Fischedick J.T., A. Hazekamp, T. Erkelens, Y. H. Choi, R. Verpoorte. Metabolic fingerprinting of Cannabis sativa L. cannabinoids and terpenoids for chemotaxonomic and drug standardization purposes. Phytochemistry 2010, 71, 2058-2073
Gong X., C. Sun, M. A. Abame, W. Shi, Y. Xie, W. Xu, F. Zhu, Y. Zhang, J. Shen, H. A. Aisa. Synthesis of CBD and its Derivatives Bearing Various C4'-Side Chains with a Late-Stage Diversification Method. J. Org. Chem. 2020, 85, 2704-2715
Gutman L.A, G. A. Nisnevich, I. Rukhman, B. Tishin, M. Etinger, I. Fedotev, B. Pertsikov, R. Khanolkar. Methods for Purifying Trans-(-)-Δ9-Tetrahydrocannabinol and Trans-(+)-Δ-Tetrahydrocannabinol. WO 2006053766 A1, 2006.
Hallow D. M., J. He, M. C. Dobish, D. Petrovic, G. Mkrtchyan. Cannabidiol Compositions Having Modified Cannabinoid Profiles. WO 2020051371 A2. 2020.
Kobayashi Y., A. Takeuchi, Y. G. Wang. Synthesis of Cannabidiols via Alkenylation of Cyclohexenyl Monoacetate. Org. Lett. 2006, 8, 2699-2702.
Lago-Fernández A., V. Redondo, L. Hernández-Folgado, L. Figuerola-Sencio, N. Jagerovic. New Method for the Synthesis of Cannabiol Derivatives. Methods in Enzymology 2017, 593, 237-257
Mechoulam R., M. Peters, E. Murillo-Rodriguez et al. Cannabidiol - recent advances. Chem. Biodivers. 2007, 4, 1678-1692
Mechoulam R., Y. Gaoni. A Total Synthesis of Δ9-Tetrahydrocannabinol, the Active Constituent of Hashish. Tetrahedron 1965, 21, 1223-1229
Palmieri, R. Ballini, P. Allegrini, D. Ciceri. Continuous Flow Synthesis of Cannabidiol. WO 2020099283 A1. 2020
Dethe D.H., R. D. Erande, S. Mahapatra, S. Das., V. Kumar B. Protecting Group Free Enantiospecific Total Syntheses of Structurally Diverse Natural Products of the Tetrahydrocannabinoid Family. Chem. Commun. 2015, 51, 2871-2873
Petrzilka T., W. Haefliger, C. Sikemeier, G. Ohloff, A. Eschenmoser. Synthese und Chiralitat des (-)-Cannabidiols Vorlaufige Mitteilung. Helv. Chim. Acta 1967, 50, 719723.28.
Petrzilka T., W. Haefliger, C. Sikemeier. Synthese von Haschisch-Inhaltsstoffen. 4. Mitteilung. Helv. Chim. Acta 1969, 52, 1102
Porco J. A., A. B. Beeler, L. E. Brown, R. V. Trilles. One-Step Flow-Mediated Synthesis of Cannabidiol (CBD) and Derivatives. U.S. 2020325091 A1. 2020.
Rickards R.W., H. Roenneberg. Synthesis of (-)-Δ9-6a,10a-trans-Tetrahydrocannabinol. Boron Trifluoride Catalyzed Arylation by a Homocuprate. J. Org. Chem. 1984, 49, 572573
Russo E.B. Taming THC: potential cannabis synergy and phytocannabinoid-terpenoid entourage effects. Br. J. Pharmacol. 2011, 163, 1344-1364
Shultz Z.P., G. A. Lawrence, J. M. Jacobson, E. J. Cruz, J. W. Leahy. Enantioselective Total Synthesis of Cannabinoids-A Route for Analogue Development. Org. Lett. 2018, 20, 381-384.
Steup C., T. Herkenroth. Process for preparing synthetic cannabinoids. US-2010298579A1.2010
Stinchcomb A.L., M. J. Golinski, J. L. Howard, D. C. Hammell, S. L. Banks. Prodrugs of Cannabidiol, Compositions Comprising Prodrugs of Cannabidiol and Methods of Using the Same. WO 2009018389 A. 2009.
Vaillancourt V., K. F. Albitazi. A One-Step Method for the o-Arylation of Camphor. Synthesis of (-)-Cannabidiol and (-)-Cannabidiol Dimethyl Ether. J. Org. Chem. 1992, 57, 3627-3631
Zhang W., Y. Li, F. Jiang. Preparation method of cannabidiol. CN 113896616 A. 2022.
Zuhse R. D., D. Ostrovskyi. Verfahren zur Herstellung von Cannabidiol. DE102019104563A1. 2020.

## Claims

1. A synthesis method for a cannabinoid compound that is selected from cannabidiol (CBD) and Δ⁹-tetrahydrocannabinol (THC), comprising the steps of:
a) dissolving olivetol in dichloromethane (DCM) with stirring;
b) adding a Lewis acid as a catalyst;
c) keeping the suspension resulting from step b) under reflux for at least 20 minutes;
d) bringing the suspension obtained in c) to room temperature;
e) adding a solution of a terpene in dichloromethane dropwise and with stirring;
f) stirring for an additional 10 to 90 minutes after finishing adding the solution from e);
g) stopping the reaction;
h) purifying the synthesized cannabinoid;
wherein the catalyst added in phase b) is FeCl₃.6H₂O.

2. The method according to claim 1, wherein the terpene that is added in step e) is selected from (+)-p-mentha-2,8-dien-1-ol, (-)-p-mentha-2,8-dien-1-ol and isopiperitenol.

3. The method according to any one of the preceding claims, wherein the ratio of equivalents of the olivetol : terpene compounds is 1 : 1 and FeCl₃.6H₂O is between 0.05-0.6 eq.

4. The method according to any one of the preceding claims, wherein the suspension resulting from step b) is kept under reflux for 20-45 minutes.

5. The method according to any one of the preceding claims, wherein the terpene that is added in step e) is at a concentration of 0.015-0.021 mmol/ml of DCM.

6. The method according to any one of the preceding claims, wherein the reaction is stopped in step g) by adding an alkaline aqueous solution.

7. The method according to claim 5, wherein the reaction is stopped in step g) by adding saturated NHCO₃.

8. The method according to claim 5 or 6, wherein the method comprises an additional step, after step g) of stopping the reaction, wherein the organic phase is separated and another additional step after that wherein the separated organic phase is washed and, prior to the purification phase, dried.

9. The method according to any one of the preceding claims, wherein the synthesized cannabinoid is purified by means of chromatography.

10. The method according to claim 9, wherein the synthesized cannabinoid is purified by means of flash column chromatography on silica.

11. The method according to claim 3, wherein the cannabinoid to be synthesized is cannabidiol and the olivetol : FeCl₃.6H₂O : terpene ratio is 1 : 0.05-0.1 : 1.

12. The method according to any one of the preceding claims, wherein the cannabinoid to be synthesized is cannabidiol and the stirring time of step f) is selected in the range of 10 to 25 minutes.

13. The method according to claim 11, wherein the cannabinoid to be synthesized is cannabidiol, and:
the selected terpene is (+)-p-mentha-2,8-dien-1-ol and the stirring time range of step f) is selected from the range of 15-25 minutes,
or
the selected terpene is isopiperitenol and the stirring time range of step f) is selected from the range of 10-20 minutes.

14. The method according to claim 12 or 13, wherein the stirring time range of step f) is 20 minutes.

15. The method according to claim 3, wherein the cannabinoid to be synthesized is Δ⁹-tetrahydrocannabinol (THC) and the olivetol : FeCl₃.6H₂O : terpene ratio is 1 : 0.5-0.6: 1.

16. The method according to any one of the preceding claims, wherein the cannabinoid to be synthesized is Δ⁹-tetrahydrocannabinol (THC) and the stirring time of step f) is selected in the range of 25 to 90 minutes.

17. The method according to claim 16, wherein the cannabinoid to be synthesized is Δ⁹-tetrahydrocannabinol (THC), and:
the selected terpene is (+)-p-mentha-2,8-dien-1-ol and the time range of step f) is selected from the range of 25-90 minutes,
or
the selected terpene is isopiperitenol and the time range of step f) is selected from the range of 20-90 minutes.

18. The method according to any one of claims 15 to 17, wherein the cannabinoid to be synthesized is Δ⁹-tetrahydrocannabinol (THC) and the stirring time of step f) is 90 minutes.

19. The method according to any one of the preceding claims, wherein:
the cannabinoid to be synthesized is cannabidiol,
the suspension resulting from step b) is kept under reflux for 20 minutes,
the terpene that is added in step e) is isopiperitenol,
the ratio of equivalents of the olivetol : FeCl₃.6H₂O : terpene compounds is 1 : 0.05 : 1,
the terpene that is added in step e) is at a concentration of 0.0165 mmol/ml of DCM,
the stirring time range of step f) is 20 minutes,
the reaction is stopped in step g) by adding saturated NHCO₃,
the method comprises an additional step, after step g) of stopping the reaction, wherein the organic phase is separated and another additional step after that wherein the separated organic phase is washed and, prior to the purification phase, dried,
the synthesized cannabidiol is purified by flash column chromatography on silica.

20. The method according to any one of the preceding claims, wherein:
the cannabinoid to be synthesized is cannabidiol,
the suspension resulting from step b) is kept under reflux for 20 minutes,
the terpene that is added in step e) is (+)-p-mentha-2,8-dien-1-ol,
the ratio of equivalents of the olivetol : FeCl₃.6H₂O : terpene compounds is 1 : 0.1 : 1,
the terpene that is added in step e) is at a concentration of 0.02 mmol/ml of DCM,
the stirring time range of step f) is 20 minutes,
the reaction is stopped in step g) by adding saturated NHCO₃,
the method comprises an additional step, after step g) of stopping the reaction, wherein the organic phase is separated and another additional step after that wherein the separated organic phase is washed and, prior to the purification phase, dried,
the synthesized cannabidiol is purified by flash column chromatography on silica.

21. The method according to any one of the preceding claims, wherein:
the cannabinoid to be synthesized is Δ⁹-tetrahydrocannabinol (THC),
the terpene that is added in step e) is isopiperitenol,
the ratio of equivalents of the olivetol : FeCl₃.6H₂O : terpene compounds is 1 : 0.6 : 1,
the terpene that is added in step e) is at a concentration of 0.024 mmol/ml of DCM,
the stirring time range of step f) is 90 minutes,
the reaction is stopped in step g) by adding saturated NHCO₃,
the method comprises an additional step, after step g) of stopping the reaction, wherein the organic phase is separated and another additional step after that wherein the separated organic phase is washed and, prior to the purification phase, dried,
the synthesized Δ⁹-tetrahydrocannabinol (THC) is purified by flash column chromatography on silica.

22. The method according to any one of the preceding claims, wherein:
the cannabinoid to be synthesized is Δ⁹-tetrahydrocannabinol (THC),
the terpene that is added in step e) is (+)-p-mentha-2,8-dien-1-ol,
the ratio of equivalents of the olivetol : FeCl₃.6H₂O : terpene compounds is 1 : 0.5 : 1,
the terpene that is added in step e) is at a concentration of 0.024 mmol/ml of DCM,
the stirring time range of step f) is 90 minutes,
the reaction is stopped in step g) by adding saturated NHCO₃,
the method comprises an additional step, after step g) of stopping the reaction, wherein the organic phase is separated and another additional step after that wherein the separated organic phase is washed and, prior to the purification phase, dried,
the synthesized Δ⁹-tetrahydrocannabinol (THC) is purified by flash column chromatography on silica.

## Patentansprüche

1. Verfahren zur Synthese einer Cannabinoidverbindung, die aus Cannabidiol (CBD) und Δ⁹-Tetrahydrocannabinol (THC) ausgewählt ist, umfassend die folgenden Schritte:
a) Lösen von Olivetol in Dichlormethan (DCM) unter Rühren;
b) Hinzufügen einer Lewis-Säure als Katalysator;
c) Halten der aus Schritt b) resultierenden Suspension unter Rückfluss für mindestens 20 Minuten;
d) Bringen der in c) erhaltenen Suspension auf Raumtemperatur;
e) Hinzufügen einer Lösung eines Terpens in Dichlormethan tropfenweise und unter Rühren;
f) Rühren für weitere 10 bis 90 Minuten nach Beendigung des Hinzufügens der Lösung aus e);
g) Beenden der Reaktion;
h) Reinigen des synthetisierten Cannabinoids;
wobei der in Phase b) hinzugefügte Katalysator FeCl₃.6H₂O ist.

2. Verfahren nach Anspruch 1, wobei das Terpen, das in Schritt e) hinzugefügt wird, ausgewählt ist aus (+)-p-Mentha-2,8-dien-1-ol, (-)-*p*-Mentha-2,8-dien-1-ol und Isopiperitenol.

3. Verfahren nach einem der vorhergehenden Ansprüche, wobei das Äquivalentverhältnis der Verbindungen Olivetol:Terpen 1:1 beträgt und FeCl₃.6H₂O zwischen 0,05-0,6 Äq. liegt.

4. Verfahren nach einem der vorhergehenden Ansprüche, wobei die aus Schritt b) resultierende Suspension für 20-45 Minuten unter Rückfluss gehalten wird.

5. Verfahren nach einem der vorhergehenden Ansprüche, wobei das Terpen, das in Schritt e) hinzugefügt wird, in einer Konzentration von 0,015-0,021 mmol/ml DCM vorliegt.

6. Verfahren nach einem der vorhergehenden Ansprüche, wobei die Reaktion in Schritt g) durch Hinzufügen einer wässrigen alkalischen Lösung beendet wird.

7. Verfahren nach Anspruch 5, wobei die Reaktion in Schritt g) durch Hinzufügen von gesättigtem NHCO₃ beendet wird.

8. Verfahren nach Anspruch 5 oder 6, wobei das Verfahren einen zusätzlichen Schritt nach dem Schritt g) des Beendens der Reaktion, wobei die organische Phase abgetrennt wird, und einen weiteren zusätzlichen Schritt danach umfasst, wobei die abgetrennte organische Phase gewaschen und vor der Reinigungsphase getrocknet wird.

9. Verfahren nach einem der vorhergehenden Ansprüche, wobei das synthetisierte Cannabinoid mittels Chromatographie gereinigt wird.

10. Verfahren nach Anspruch 9, wobei das synthetisierte Cannabinoid mittels Flash-Säulenchromatographie auf Silica gereinigt wird.

11. Verfahren nach Anspruch 3, wobei das zu synthetisierende Cannabinoid Cannabidiol und das Verhältnis Olivetol:FeCl₃.6H₂O:Terpen 1:0,05-0,1:1 ist.

12. Verfahren nach einem der vorhergehenden Ansprüche, wobei das zu synthetisierende Cannabinoid Cannabidiol ist und die Rührzeit von Schritt f) im Bereich von 10 bis 25 Minuten gewählt wird.

13. Verfahren nach Anspruch 11, wobei das zu synthetisierende Cannabinoid Cannabidiol ist, und:
das ausgewählte Terpen (+)-*p*-Mentha-2,8-dien-1-ol ist und der Rührzeitbereich von Schritt f) aus dem Bereich von 15-25 Minuten ausgewählt ist,
oder
das ausgewählte Terpen Isopiperitenol ist und der Rührzeitbereich von Schritt f) aus dem Bereich von 10-20 Minuten ausgewählt ist.

14. Verfahren nach Anspruch 12 oder 13, wobei der Rührzeitbereich von Schritt f) 20 Minuten ist.

15. Verfahren nach Anspruch 3, wobei das zu synthetisierende Cannabinoid Δ⁹-Tetrahydrocannabinol (THC) und das Verhältnis Olivetol:FeCl₃.6H₂O:Terpen 1:0,5-0,6:1 ist.

16. Verfahren nach einem der vorhergehenden Ansprüche, wobei das zu synthetisierende Cannabinoid Δ⁹-Tetrahydrocannabinol (THC) ist und die Rührzeit von Schritt f) im Bereich von 25 bis 90 Minuten gewählt wird.

17. Verfahren nach Anspruch 16, wobei das zu synthetisierende Cannabinoid Δ⁹-Tetrahydrocannabinol (THC) ist, und:
das ausgewählte Terpen (+)-*p*-Mentha-2,8-dien-1-ol ist und der Zeitbereich von Schritt f) aus dem Bereich von 25-90 Minuten ausgewählt ist,
oder
das ausgewählte Terpen Isopiperitenol ist und der Zeitbereich von Schritt f) aus dem Bereich von 20-90 Minuten ausgewählt ist.

18. Verfahren nach einem der Ansprüche 15 bis 17, wobei das zu synthetisierende Cannabinoid Δ⁹-Tetrahydrocannabinol (THC) ist und die Rührzeit von Schritt f) 90 Minuten beträgt.

19. Verfahren nach einem der vorhergehenden Ansprüche, wobei:
das zu synthetisierende Cannabinoid Cannabidiol ist,
die aus Schritt b) resultierende Suspension für 20 Minuten unter Rückfluss gehalten wird,
das Terpen, das in Schritt e) hinzugefügt wird, Isopiperitenol ist,
das Äquivalentverhältnis der Verbindungen Olivetol:FeCl₃.6H₂O:Terpen 1:0,05:1 beträgt,
das Terpen, das in Schritt e) hinzugefügt wird, in einer Konzentration von 0,0165 mmol/ml DCM vorliegt,
der Rührzeitbereich von Schritt f) 20 Minuten beträgt,
die Reaktion in Schritt g) durch Hinzufügen von gesättigtem NHCO₃ beendet wird,
das Verfahren einen zusätzlichen Schritt nach dem Schritt g) des Beendens der Reaktion, wobei die organische Phase abgetrennt wird, und einen weiteren zusätzlichen Schritt danach umfasst, wobei die abgetrennte organische Phase gewaschen und vor der Reinigungsphase getrocknet wird,
das synthetisierte Cannabidiol durch Flash-Säulenchromatographie auf Silica gereinigt wird.

20. Verfahren nach einem der vorhergehenden Ansprüche, wobei:
das zu synthetisierende Cannabinoid Cannabidiol ist,
die aus Schritt b) resultierende Suspension für 20 Minuten unter Rückfluss gehalten wird,
das Terpen, das in Schritt e) hinzugefügt wird, (+)-*p*-Mentha-2,8-dien-1-ol ist,
das Äquivalentverhältnis der Verbindungen Olivetol:FeCl₃.6H₂O:Terpen 1:0,1:1 beträgt,
das Terpen, das in Schritt e) hinzugefügt wird, in einer Konzentration von 0,02 mmol/ml DCM vorliegt,
der Rührzeitbereich von Schritt f) 20 Minuten beträgt,
die Reaktion in Schritt g) durch Hinzufügen von gesättigtem NHCO₃ beendet wird,
das Verfahren einen zusätzlichen Schritt nach dem Schritt g) des Beendens der Reaktion, wobei die organische Phase abgetrennt wird, und einen weiteren zusätzlichen Schritt danach umfasst, wobei die abgetrennte organische Phase gewaschen und vor der Reinigungsphase getrocknet wird,
das synthetisierte Cannabidiol durch Flash-Säulenchromatographie auf Silica gereinigt wird.

21. Verfahren nach einem der vorhergehenden Ansprüche, wobei:
das zu synthetisierende Cannabinoid Δ⁹-Tetrahydrocannabinol (THC) ist, das Terpen, das in Schritt e) hinzugefügt wird, Isopiperitenol ist,
das Äquivalentverhältnis der Verbindungen Olivetol:FeCl₃.6H₂O:Terpen 1:0,6:1 beträgt,
das Terpen, das in Schritt e) hinzugefügt wird, in einer Konzentration von 0,024 mmol/ml DCM vorliegt,
der Rührzeitbereich von Schritt f) 90 Minuten beträgt,
die Reaktion in Schritt g) durch Hinzufügen von gesättigtem NHCO₃ beendet wird,
das Verfahren einen zusätzlichen Schritt nach dem Schritt g) des Beendens der Reaktion, wobei die organische Phase abgetrennt wird, und einen weiteren zusätzlichen Schritt danach umfasst, wobei die abgetrennte organische Phase gewaschen und vor der Reinigungsphase getrocknet wird,
das synthetisierte Δ⁹-Tetrahydrocannabinol (THC) durch Flash-Säulenchromatographie auf Silica gereinigt wird.

22. Verfahren nach einem der vorhergehenden Ansprüche, wobei:
das zu synthetisierende Cannabinoid Δ⁹-Tetrahydrocannabinol (THC) ist, das Terpen, das in Schritt e) hinzugefügt wird, (+)-*p*-Mentha-2,8-dien-1-ol ist,
das Äquivalentverhältnis der Verbindungen Olivetol:FeCl₃.6H₂O:Terpen 1:0,5:1 beträgt,
das Terpen, das in Schritt e) hinzugefügt wird, in einer Konzentration von 0,024 mmol/ml DCM vorliegt,
der Rührzeitbereich von Schritt f) 90 Minuten beträgt,
die Reaktion in Schritt g) durch Hinzufügen von gesättigtem NHCO₃ beendet wird,
das Verfahren einen zusätzlichen Schritt nach dem Schritt g) des Beendens der Reaktion, wobei die organische Phase abgetrennt wird, und einen weiteren zusätzlichen Schritt danach umfasst, wobei die abgetrennte organische Phase gewaschen und vor der Reinigungsphase getrocknet wird,
das synthetisierte Δ⁹-Tetrahydrocannabinol (THC) durch Flash-Säulenchromatographie auf Silica gereinigt wird.

## Revendications

1. Procédé de synthèse d'un composé cannabinoïde qui est choisi parmi le cannabidiol (CBD) et le Δ⁹-tétrahydrocannabinol (THC), comprenant les étapes consistant à :
a) dissoudre de l'olivétol dans le dichlorométhane (DCM) sous agitation ;
b) ajouter un acide de Lewis comme catalyseur;
c) maintenir la suspension résultant de l'étape b) à reflux pendant au moins 20 minutes ;
d) porter la suspension obtenue dans c) à la température ambiante ;
e) ajouter une solution d'un terpène dans le dichlorométhane goutte à goutte et sous agitation ;
f) agiter pendant 10 à 90 minutes supplémentaires après avoir fini d'ajouter la solution de l'étape e) ;
g) arrêter la réaction ;
h) purifier le cannabinoïde synthétisé ;
dans lequel le catalyseur ajouté dans la phase b) est FeCl₃.6H₂O.

2. Procédé selon la revendication 1, dans lequel le terpène qui est ajouté à l'étape e) est choisi parmi (+)-p-mentha-2,8-diène-1-ol, (-)-p-mentha-2,8-dien-1-ol et isopipérénol.

3. Procédé selon l'une quelconque des revendications précédentes, dans lequel le rapport d'équivalents des composés olivétol:terpène est de 1:1 et la quantité de FeCl₃.6H₂O est entre 0,05 et 0,6 éq.

4. Procédé selon l'une quelconque des revendications précédentes, dans lequel la suspension résultant de l'étape b) est maintenue à reflux pendant 20 à 45 minutes.

5. Procédé selon l'une quelconque des revendications précédentes, dans lequel le terpène qui est ajouté à l'étape e) est à une concentration de 0,015-0,021 mmol/mL de DCM.

6. Procédé selon l'une quelconque des revendications précédentes, dans lequel la réaction est arrêtée à l'étape g) par l'ajout d'une solution aqueuse alcaline.

7. Procédé selon la revendication 5, dans lequel la réaction est arrêtée à l'étape g) par l'ajout de NHCO₃saturé.

8. Procédé selon la revendication 5 ou 6, dans lequel le procédé comprend une étape supplémentaire, après l'étape g) d'arrêt de la réaction, dans laquelle la phase organique est séparée suivie d'une autre étape supplémentaire dans laquelle la phase organique séparée est lavée et, avant la phase de purification, séchée.

9. Procédé selon l'une quelconque des revendications précédentes, dans lequel le cannabinoïde synthétisé est purifié par chromatographie.

10. Procédé selon la revendication 9, dans lequel le cannabinoïde synthétisé est purifié par chromatographie flash sur colonne de silice.

11. Procédé selon la revendication 3, dans lequel le cannabinoïde à synthétiser est le cannabidiol et le rapport olivétol:FeCl₃.6H₂O:terpène est de 1:0,05-0,1:1.

12. Procédé selon l'une quelconque des revendications précédentes, dans lequel le cannabinoïde à synthétiser est le cannabidiol et la durée d'agitation de l'étape f) est choisie dans la plage de 10 à 25 minutes.

13. Procédé selon la revendication 11, dans lequel le cannabinoïde à synthétiser est le cannabidiol, et :
le terpène choisi est le (+)-p-mentha-2,8-dien-1-ol et la plage de durée d'agitation de l'étape f) est choisie dans la plage de 15 à 25 minutes,
ou
le terpène choisi est l'isopipériténol et la plage de durée d'agitation de l'étape f) est choisie dans la plage de 10 à 20 minutes.

14. Procédé selon la revendication 12 ou 13, dans lequel la plage de durée d'agitation de l'étape f) est de 20 minutes.

15. Procédé selon la revendication 3, dans lequel le cannabinoïde à synthétiser est le Δ⁹-tétrahydrocannabinol (THC) et le rapport olivétol:FeCl₃.6H₂O:terpène est de 1:0,5-0,6:1.

16. Procédé selon l'une quelconque des revendications précédentes, dans lequel le cannabinoïde à synthétiser est le Δ⁹-tétrahydrocannabinol (THC) et la durée d'agitation de l'étape f) est choisie dans la plage de 25 à 90 minutes.

17. Procédé selon la revendication 16, dans lequel le cannabinoïde à synthétiser est le Δ⁹-tétrahydrocannabinol (THC), et :
le terpène choisi est le (+)-p-mentha-2,8-dien-1-ol et la plage de durée de l'étape f) est choisie dans la plage de 25 à 90 minutes,
ou
le terpène choisi est l'isopipériténol et la plage de durée de l'étape f) est choisie dans la plage de 20 à 90 minutes.

18. Procédé selon l'une quelconque des revendications 15 à 17, dans lequel le cannabinoïde à synthétiser est le Δ⁹-tétrahydrocannabinol (THC) et la durée d'agitation de l'étape f) est de 90 minutes.

19. Procédé selon l'une quelconque des revendications précédentes, dans lequel :
le cannabinoïde à synthétiser est le cannabidiol,
la suspension résultant de l'étape b) est maintenue à reflux pendant 20 minutes,
le terpène qui est ajouté à l'étape e) est l'isopipérénol,
le rapport d'équivalents des composés olivétol:FeCl₃.6H₂O:terpène est de 1:0,05:1,
le terpène qui est ajouté à l'étape e) est à une concentration de 0,0165 mmol/mL de DCM,
la plage de durée d'agitation de l'étape f) est de 20 minutes,
la réaction est arrêtée à l'étape g) par l'ajout de NHCO₃ saturé,
le procédé comprend une étape supplémentaire, après l'étape g) d'arrêt de la réaction,
dans laquelle la phase organique est séparée suivie d'une autre étape supplémentaire dans laquelle la phase organique séparée est lavée et, avant la phase de purification, séchée,
le cannabidiol synthétisé est purifié par chromatographie flash sur colonne de silice.

20. Procédé selon l'une quelconque des revendications précédentes, dans lequel :
le cannabinoïde à synthétiser est le cannabidiol,
la suspension résultant de l'étape b) est maintenue à reflux pendant 20 minutes,
le terpène qui est ajouté à l'étape e) est le (+)-p-mentha-2,8-diène-1-ol,
le rapport d'équivalents des composés olivétol:FeCl₃.6H₂O:terpène est de 1:0,1:1,
le terpène qui est ajouté à l'étape e) est à une concentration de 0,02 mmol/mL de DCM,
la plage de durée d'agitation de l'étape f) est de 20 minutes,
la réaction est arrêtée à l'étape g) par l'ajout de NHCO₃ saturé,
le procédé comprend une étape supplémentaire, après l'étape g) d'arrêt de la réaction,
dans laquelle la phase organique est séparée suivie d'une autre étape supplémentaire dans laquelle la phase organique séparée est lavée et, avant la phase de purification, séchée,
le cannabidiol synthétisé est purifié par chromatographie flash sur colonne de silice.

21. Procédé selon l'une quelconque des revendications précédentes, dans lequel :
le cannabinoïde à synthétiser est le Δ⁹-tétrahydrocannabinol (THC), le terpène qui est ajouté à l'étape e) est l'isopipérénol,
le rapport d'équivalents des composés olivétol:FeCl₃.6H₂O:terpène est de 1:0,6:1,
le terpène qui est ajouté à l'étape e) est à une concentration de 0,024 mmol/mL de DCM,
la plage de durée d'agitation de l'étape f) est de 90 minutes,
la réaction est arrêtée à l'étape g) par l'ajout de NHCO₃ saturé,
le procédé comprend une étape supplémentaire, après l'étape g) d'arrêt de la réaction,
dans laquelle la phase organique est séparée suivie d'une autre étape supplémentaire dans laquelle la phase organique séparée est lavée et, avant la phase de purification, séchée,
le Δ⁹-tétrahydrocannabinol (THC) synthétisé est purifié par chromatographie flash sur colonne de silice.

22. Procédé selon l'une quelconque des revendications précédentes, dans lequel :
le cannabinoïde à synthétiser est le Δ⁹-tétrahydrocannabinol (THC), le terpène qui est ajouté à l'étape e) est le (+)-p-mentha-2,8-diène-1-ol,
le rapport d'équivalents des composés olivétol:FeCl₃.6H₂O:terpène est de 1:0,5:1,
le terpène qui est ajouté à l'étape e) est à une concentration de 0,024 mmol/mL de DCM,
la plage de durée d'agitation de l'étape f) est de 90 minutes,
la réaction est arrêtée à l'étape g) par l'ajout de NHCO₃ saturé,
le procédé comprend une étape supplémentaire, après l'étape g) d'arrêt de la réaction,
dans laquelle la phase organique est séparée suivie d'une autre étape supplémentaire dans laquelle la phase organique séparée est lavée et, avant la phase de purification, séchée,
le Δ⁹-tétrahydrocannabinol (THC) synthétisé est purifié par chromatographie flash sur colonne de silice.
